# EUROPEAN PATENT APPLICATION

(11) **EP 4 212 170 A1**
(43) Date of publication of application: **19.07.2023**
(21) Application number: 22206545.0
(22) Date of filing: 20.08.2019
(51) Int. Cl.: A61K 38/20, A61K 39/395, A61K 9/00, A61P 27/02

(54) **NON-LEAKING OR MINIMALLY-LEAKING CHOROIDAL OR RETINAL REVASCULARIZATION**

(30) Priority: 21.08.2018 US 201862720441 P
(62) Divisional of application: 19852434.0
(71) Applicant: California Institute of Technology, Pasadena, CA 91125 (US); The Regents of The University of California, Oakland CA 94607-5200 (US); United States Government as Represented by the Department of Veterans Affairs, Washington, District of Columbia 20420 (US)
(72) Inventor: GRUBBS, Robert, H., Pasadena, CA 91125 (US); SCHWARTZ, Daniel, M., OAKLAND, CA 94607 (US)
(74) Representative: Murgitroyd & Company

(57) **Abstract**

Disclosed herein include methods, kits, formulations, and compositions for increasing choroidal or retinal perfusion or promoting non-leaking or minimally-leaking choroidal or retinal revascularization in a subject in need thereof. An effective amount of an angiogenesis factor (e.g., a pro-angiogenic factor and/or a vascular maturation factor) can be administered to the subject.

## Description

The present application claims priority under 35 U.S.C. § 119(e) to U.S. Application No. 62/720,441, filed August 21, 2018. The content of the related application is incorporated herein by reference in its entirety.

### BACKGROUND

### Field

The present disclosure relates generally to the field of treating ocular diseases, for example by increasing non-leaking or minimally leaking choroidal or retinal revascularization.

### Description of the Related Art

Age-related macular degeneration (AMD) is the leading cause of central visual loss in the developed world. The "dry" form of the disease is characterized by yellow deposits (drusen), which accumulate underneath the retinal pigment epithelium (RPE). In some cases of dry AMD, there is progressive atrophy of the RPE, overlying photoreceptors, and subjacent choriocapillaris. This "geographic atrophy" (GA) often extends progressively around the macular center (fovea), until it finally involves the fovea and causes irreversible central visual loss. In other cases, patients with dry AMD develop choroidal neovascularization (CNV) that preferentially grows underneath the RPE. These abnormal vessels leak fluid and blood, which causes scar tissue, or a disciform scar, to replace the normal macular tissue. Loss of normal macular tissue in wet AMD also causes irreversible visual loss.

### SUMMARY

Disclosed herein include embodiments of a method for increasing choroidal perfusion in a subject in need thereof. In some embodiments, the method comprises: administering a formulation comprising a therapeutically effective amount of a pro-angiogenic factor and/or a vascular maturation factor to the subject in need of increased choroidal perfusion. In some embodiments, the method results in increasing non-leaking or minimally-leaking choroidal perfusion in the subject. Disclosed herein include embodiments of a method for promoting non-leaking or minimally-leaking choroidal revascularization in a subject in need thereof. In some embodiments, the method comprises: administering a formulation comprising a therapeutically effective amount of a pro-angiogenic factor and/or a vascular maturation factor to the subject in need of non-leaking or minimally-leaking choroidal revascularization. In some embodiments, the method results in promoting non-leaking or minimally-leaking choroidal revascularization in the subject.

Disclosed herein include embodiments of a method for increasing choroidal perfusion in a subject in need thereof. In some embodiments, the method comprises: administering a formulation comprising a therapeutically effective amount of an angiogenesis factor to the subject in need of increased choroidal perfusion. In some embodiments, the method results in increasing non-leaking or minimally-leaking choroidal perfusion in the subject. Disclosed herein include embodiments of a method for promoting non-leaking or minimally-leaking choroidal revascularization in a subject in need thereof. In some embodiments, the method comprises: administering a formulation comprising a therapeutically effective amount of an angiogenesis factor to the subject in need of non-leaking or minimally-leaking choroidal revascularization. In some embodiments, the method results in promoting non-leaking or minimally-leaking choroidal revascularization in the subject. The angiogenesis factor can comprise, or can be, a pro-angiogenic factor and/or a vascular maturation factor.

Disclosed herein include embodiments of a method for increasing choroidal perfusion in a subject in need thereof. In some embodiments, the method comprises: causing a level of a pro-angiogenic factor in the choroid of an eye of the subject to increase; and administering a therapeutically effective amount of a vascular maturation factor to the subject. In some embodiments, the method results in increasing non-leaking or minimally-leaking choroidal perfusion in the subject. Disclosed herein include embodiments of a method for promoting non-leaking or minimally-leaking choroidal revascularization in a subject in need thereof. In some embodiments, the method, comprises: causing a level of a pro-angiogenic factor in the choroid of an eye of the subject to increase; and administering a therapeutically effective amount of a vascular maturation factor to the subject. In some embodiments, the method results in promoting non-leaking or minimally-leaking choroidal revascularization in the subject. Causing the level of the pro-angiogenic factor in the choroid of the eye of the subject to increase can comprise discontinuing or reducing the administration frequency of an antagonist of a second pro-angiogenic factor. The pro-angiogenic factor and the second pro-angiogenic factor can be identical, or different. The pro-angiogenic factor and the second pro-angiogenic factor can be different. The antagonist of the second pro-angiogenic factor can comprise an antibody targeting the second pro-angiogenic factor, or a fragment thereof. Causing the level of the second pro-angiogenic factor in the choroid of the eye of the subject to increase can comprise administering a therapeutically effective amount of the second pro-angiogenic factor to the subject.

In some embodiments, macular flow voids are reduced, hypoxia in the outer retina and retinal pigment epithelium (RPE) of the eye of the subject is reduced, and/or ischemia in the outer retina and RPE of the eye of the subject is reduced. In some embodiments. In some embodiments, the method results in increasing choroidal perfusion in the subject.

In some embodiments, the method comprises: determining an extent of choroidal perfusion or non-leaking or minimally-leaking choroidal revascularization in the subject to be inadequate; and continuing to administer the formulation comprising the effective amount of the pro-angiogenic factor and/or the vascular maturation factor to the subject. The determining can comprise performing an ocular examination or sequential ocular examinations. The sequential ocular examinations can comprise visual acuity assessment, a fundus auto-fluorescence (FAF) examination, an optical coherence tomography (OCT) examination, an optical coherence tomography angiography (OCT-A) examination, a fluorescein angiography (FA) examination, an indocyanine green (ICG) angiography examination, or a combination thereof.

In some embodiments, the method comprises, prior to the administering, determining whether the subject is in need of increased choroidal perfusion or non-leaking or minimally-leaking choroidal revascularization with an ocular examination. In some embodiments, the subject has a disease that is dry age-related macular degeneration (AMD) and/or geographic atrophy (GA), or a combination thereof. The method can thereby result in reversing, halting, or slowing the progression of the disease. The reversing, halting or slowing of the progression of the disease can be mediated by increased choroidal perfusion and/or non-leaking or minimally-leaking choroidal revascularization.

In some embodiments, the subject has a disease that is wet age-related macular degeneration (AMD), choroidal neovascularization (CNV), polypoidal choroidal vasculopathy, degenerative (pathologic) myopia, or a combination thereof. The method can thereby result in reversing, halting, or slowing the progression of the disease. The reversing, halting, or slowing of the progression of the disease can be mediated by increased choroidal perfusion and/or non-leaking or minimally-leaking choroidal revascularization. In some embodiments, the method comprises administering a therapeutically effective amount of an antagonist of a second pro-angiogenic factor that reduces vascular leakage while non-leaking or minimally-leaking choroidal neovascularization develops.

Disclosed herein include embodiments of a method for increasing retinal perfusion in a subject in need thereof. In some embodiments, the method comprises: administering a formulation comprising a therapeutically effective amount of a pro-angiogenic factor and/or a vascular maturation factor to the subject in need of increased retinal perfusion. In some embodiments, the method results in increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: administering a formulation comprising a therapeutically effective amount of a pro-angiogenic factor and/or a vascular maturation factor to the subject in need of non-leaking or minimally-leaking retinal revascularization. In some embodiments, the method results in promoting non-leaking or minimally-leaking retinal revascularization in the subj ect.

Disclosed herein include embodiments of a method for increasing retinal perfusion in a subject in need thereof. In some embodiments, the method comprises: administering a formulation comprising a therapeutically effective amount of an angiogenesis factor to the subject in need of increased retinal perfusion. In some embodiments, the method results in increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: administering a formulation comprising a therapeutically effective amount of an angiogenesis factor to the subject in need of non-leaking or minimally-leaking retinal revascularization. In some embodiments, the method results in promoting non-leaking or minimally-leaking retinal revascularization in the subject. The angiogenesis factor can comprise, or can be, a pro-angiogenic factor and/or a vascular maturation factor.

Disclosed herein include embodiments of a method for increasing retinal perfusion in a subject in need thereof. In some embodiments, the method comprises: causing a level of a pro-angiogenic factor in the retina of an eye of the subject in need of increased retinal perfusion to increase; and administering a therapeutically effective amount of a vascular maturation factor to the subject. In some embodiments, the method results in increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: causing a level of a pro-angiogenic factor in the retina of an eye of the subject in need of non-leaking or minimally-leaking retinal revascularization to increase; and administering a therapeutically effective amount of a vascular maturation factor to the subject. In some embodiments, the method results in promoting non-leaking or minimally-leaking retinal revascularization in the subject. In some embodiments, causing the level of the pro-angiogenic factor in the retina of the eye of the subject to increase comprises discontinuing or reducing the administration frequency of an antagonist of a second pro-angiogenic factor. The pro-angiogenic factor and the second pro-angiogenic factor can be identical, or different. The antagonist of the second pro-angiogenic factor can comprise an antibody targeting the second pro-angiogenic factor, or a fragment thereof. In some embodiments, causing the level of the pro-angiogenic factor in the retina of the eye of the subject to increase comprises administering a therapeutically effective amount of the pro-angiogenic factor to the subject.

In some embodiments, the method results in hypoxia in the retina of the eye of the subject is reduced, and/or ischemia in the retina of the eye of the subject is reduced. In some embodiments, the method thereby results in increasing non-leaking or minimally-leaking retinal perfusion in the subject.

In some embodiments, the method comprises: determining an extent of retinal perfusion or non-leaking or minimally-leaking retinal revascularization in the subject to be inadequate; and continue administering the formulation to the subject. The determining can comprise performing an ocular examination or sequential ocular examinations. The ocular examination or the sequential ocular examinations can comprise visual acuity assessment, a fundus auto-fluorescence (FAF) examination, an optical coherence tomography (OCT) examination, an optical coherence tomography angiography (OCT-A) examination, a fluorescein angiography (FA) examination, an indocyanine green (ICG) angiography examination, or a combination thereof.

In some embodiments, the method comprises, prior to the administering, determining the subject is in need of increased non-leaking or minimally-leaking retinal perfusion or non-leaking or minimally-leaking retinal revascularization with an ocular examination. In some embodiments, the subject has a disease that is diabetic macular edema, macular edema from retinal vein occlusion, diabetic retinopathy, retinal vein occlusion, retinopathy of prematurity, retinal neovascularization in diabetes, optic nerve neovascularization in diabetes, familial exudative vitreoretinopathy, sickle cell disease, or a combination thereof. The method can thereby result in halting or slowing the progression of the disease. The halting or slowing of the progression of the disease can be mediated by increased non-leaking or minimally-leaking retinal perfusion and/or non-leaking or minimally-leaking retinal revascularization. The method can comprise administering a therapeutically effective amount of an antagonist of a second pro-angiogenic factor.

In some embodiments, the subject has a disease that is radiation retinopathy, radiation optic neuropathy, or a combination thereof. The method can thereby result in reversing, halting, or slowing the progression of the disease. The reversing, halting or slowing of the progression of the disease can be mediated by increased non-leaking or minimally-leaking retinal perfusion, non-leaking or minimally-leaking retinal revascularization and/or non-leaking or minimally-leaking revascularization of the optic nerve of the eye of the subject. The subject can have received a radiation treatment for a disease that is an intraocular tumor a head tumor, a neck tumor, or a combination thereof, resulting in delayed onset of the disease. The administering can comprise administering the formulation comprising the effective amount of the pro-angiogenic factor and/or the vascular maturation factor about 1-26 weeks after the subject receives the radiation treatment.

Disclosed herein include embodiments of a method for treating an ocular disease in a subject in need thereof. In some embodiments, the method comprises: administering a formulation to the subject, wherein the formulation comprises a therapeutically effective amount of a pro-angiogenic factor and/or a vascular maturation factor. In some embodiments, the method results in treating or slowing the progression of the ocular disease in the subject. Disclosed herein include embodiments of a method for treating an ocular disease in a subject in need thereof. In some embodiments, the method comprises: causing a level of a pro-angiogenic factor in the choroid or retina of an eye of the subject to increase; and administering a therapeutically effective amount of a vascular maturation factor to the subject, treating or slowing the progression of the ocular disease in the subject. In some embodiments, causing the level of the pro-angiogenic factor in the choroid of the eye of the subject to increase comprises discontinuing or reducing the administration frequency of an antagonist of a second pro-angiogenic factor. The pro-angiogenic factor and the second pro-angiogenic factor can be identical. The antagonist of the second pro-angiogenic factor can comprise an antibody targeting the second pro-angiogenic factor, or a fragment thereof. In some embodiments, causing the level of the pro-angiogenic factor in the choroid of the eye of the subject to increase comprises administering a therapeutically effective amount of the pro-angiogenic factor to the subject.

In some embodiments, the ocular disease is wet age-related macular degeneration (AMD), polypoidal choroidal vasculopathy (PCV), degenerate (pathologic) myopia, or a combination thereof. In some embodiments, the ocular disease is associated with, or characterized by, choroidal hypoperfusion, choroidal neovascularization (CNV), macular atrophy, or a combination thereof. In some embodiments, the ocular disease is dry age-related macular degeneration (AMD), diabetic macular edema, macular edema from retinal vein occlusion, diabetic retinopathy, retinal vein occlusion, retinopathy of prematurity, retinal neovascularization in diabetes, optic nerve neovascularization in diabetes, familial exudative vitreoretinopathy, radiation retinopathy, radiation optic neuropathy, sickle cell disease, or a combination thereof. In some embodiments, the ocular disease is associated with, or characterized by, retinal hypoperfusion, retinal ischemia, optic nerve ischemia, or a combination thereof.

In some embodiments, the method comprises determining the severity of the ocular disease in the subject or the rate of progression of the ocular disease in the subject. In some embodiments, the method comprises identifying the subject as needing increased choroidal perfusion, as needing non-leaking or minimally-leaking choroidal revascularization, as needing increased retinal perfusion, as needing non-leaking or minimally-leaking retinal revascularization, as suffering from the ocular disease, or a combination thereof. In some embodiments, the subject is known to need increased choroidal perfusion, non-leaking or minimally-leaking choroidal revascularization, increased retinal perfusion, non-leaking or minimally-leaking retinal revascularization, or have the ocular disease.

In some embodiments, the pro-angiogenic factor is, or comprises, a recombinant pro-angiogenic factor, a mutant pro-angiogenic factor, a fragment of the pro-angiogenic factor, or a combination thereof. The pro-angiogenic factor can be, or can comprise, vascular endothelial growth factor (VEGF), angiopoietin-2 (Ang-2), or a combination thereof. The VEGF can be, or can comprise, VEGF-A, VEGF-B, VEGF-C, VEGF-D, placental growth factor (PIGF), or a combination thereof. In some embodiments, the vascular maturation factor is, or comprises, a recombinant vascular maturation factor, a mutant vascular maturation factor, a fragment of the vascular maturation factor, or a combination thereof. The vascular maturation factor can be, or can comprise, platelet-derived growth factor (PDGF), angiopoietin-1 (Ang-1), or a combination thereof. The vascular maturation factor can be, or can comprise, PDGF subunit A, PDGF subunit B, PDGF subunit C, PDGF subunit D, or a combination thereof.

In some embodiments, thereby exudation or neovascularization in the choroid or retina of the eye of the subject is reduced. The method can comprise determining the exudation in the choroid or retina of the eye of the subject is reduced using optical coherence tomography (OCT). The method can comprise determining the neovascularization in the choroid or retina of the eye of the subject is reduced using optical coherence tomography angiography (OCT-A), fluorescein angiography (FA), indocyanine green (ICG) angiography, or a combination thereof. In some embodiments, thereby non-leaking or minimally-leaking choroidal perfusion and/or non-leaking and/or minimally-leaking retinal perfusion increases in the subject by at least 5%.

In some embodiments, thereby new non-leaking or minimally-leaking blood vessels are formed in the choroid or the retina of the eye of the subject. The method can comprise determining the formation of new blood vessels using optical coherence tomography angiography (OCT-A). The method can comprise determining minimal or no exudation from the new blood vessels is minimal using optical coherence tomography (OCT), which indicates the new blood vessels formed are non-leaking or minimally-leaking. The new non-leaking or minimally-leaking blood vessels formed in the choroid or the retina of the eye of the subject can cover at least 5% of the macular region of the eye of the subject. The new non-leaking or minimally-leaking blood vessels formed in the choroid or retina of the eye of the subject can cover at least 5% of the peripheral choroid or peripheral retina of the eye of the subject. The visual acuity of the subject can stabilize or improve. Choroidal hypoxia and/or retinal hypoxia can be mitigated in the subject.

In some embodiments, thereby a hypoxia inducible factor (HIF)-mediated blinding complication is mitigated. The HIF-mediated visual loss complication can comprise choroidal neovascularization (CNV), retinal neovascularization, macular edema, or a combination thereof. In some embodiments, thereby retinal edema, subretinal fluid, or both, are reduced. In some embodiments, thereby leaky choroidal neovascularization is mitigated in the subject. In some embodiments, macular atrophy, geographic atrophy (GA), or both are mitigated in the subj ect.

In some embodiments, the administering comprises administering the formulation intravitreally. The administering can comprise administering the formulation subretinally. The administering can comprise administering the formulation to the suprachoroidal space of an eye of the subject. The administering can comprise administering the formulation to the macular region of an eye of the subject. The administering can comprise administering the formulation to one or more retinal or choroidal regions, of the eye of the subject, with reduced perfusion. The administering can comprise administering the formulation to the subject about once every week to about once every year. The administering can comprise administering the formulation over about three months to about 12 months.

In some embodiments, the method comprises: using optical coherence tomography angiography (OCT-A) and optical coherence tomography (OCT) to determine choroidal or retinal revascularization or non-leaking or minimally-leaking choroidal or retinal revascularization in the subject, respectively; and if adequate, discontinuing temporarily or permanently administering the formulation to the subject. In some embodiments, the method comprises: using optical coherence tomography angiography (OCT-A) and optical coherence tomography (OCT) to determine choroidal or retinal revascularization or non-leaking or minimally-leaking choroidal or retinal revascularization in the subject, respectively; and if inadequate, continuing administering the formulation to the subject. In some embodiments, the method comprises: using optical coherence tomography angiography (OCT-A) and optical coherence tomography (OCT) to determine choroidal or retinal revascularization or non-leaking or minimally-leaking choroidal or retinal revascularization in the subject, respectively; and if excessive, administering an antagonist of a second pro-angiogenic factor to the subject. The antagonist of the second pro-angiogenic factor can comprise an antibody targeting the second pro-angiogenic factor. The determining can comprise performing an ocular examination or sequential ocular examinations. The sequential ocular examinations can comprise visual acuity assessment, a fundus auto-fluorescence (FAF) examination, an optical coherence tomography (OCT) examination, an optical coherence tomography angiography (OCT-A) examination, a fluorescein angiography (FA) examination, indocyanine green (ICG) angiography examination, or a combination thereof. In some embodiments, the method comprises: determining vascular maturation in the subject using optical coherence tomography angiography (OCT), fluorescein angiography (FA), indocyanine green (ICG) angiography, or a combination thereof; and if inadequate, administering the vascular maturation factor to the subject. The determining can comprise determining the extent of vascular maturation using optical coherence tomography (OCT) or fluorescein and indocyanine green (ICG) angiography. The method can comprise: using optical coherence tomography angiography (OCT-A) to determine choroidal revascularization in the subject.

In some embodiments, the therapeutically effective amount of the pro-angiogenic factor is about 0.01 mg to about 100 mg per administering. The therapeutically effective amount of the vascular maturation factor can be about 0.01 mg to about 200 mg per administering. The formulation can comprise about 0.001 mg/ml to about 200 mg/ml of the pro-angiogenic factor. The formulation can comprise about 0.001 mg/ml to about 200 mg/ml of the vascular maturation factor. The formulation can comprise a sustained release formulation of the pro-angiogenic factor and the vascular maturation factor.

Disclosed herein include embodiments of a composition comprising a pro-angiogenic factor and/or a vascular maturation factor for use in increasing non-leaking or minimally-leaking choroidal perfusion or non-leaking or minimally-leaking retinal perfusion in a subject in need thereof. Disclosed herein include embodiments of a composition comprising a pro-angiogenic factor and/or a vascular maturation factor for use in the treatment of an ocular disease associated with, or characterized by, choroidal hypoperfusion or retinal hypoperfusion in a subject in need thereof. Disclosed herein include embodiments of a composition comprising an angiogenesis factor for use in increasing non-leaking or minimally-leaking choroidal perfusion or non-leaking or minimally-leaking retinal perfusion in a subject in need thereof. Disclosed herein include embodiments of a composition comprising an angiogenesis for use in the treatment of an ocular disease associated with, or characterized by, choroidal hypoperfusion or retinal hypoperfusion in a subject in need thereof. The angiogenesis factor can be a pro-angiogenic factor and/or a vascular maturation factor.

In some embodiments, the pro-angiogenic factor is, or comprises, a recombinant pro-angiogenic factor, a mutant pro-angiogenic factor, a fragment of the pro-angiogenic factor, or a combination thereof. The pro-angiogenic factor can be, or can comprise, vascular endothelial growth factor (VEGF), angiopoietin-2 (Ang-2), or a combination thereof. The VEGF can be, or can comprise, VEGF-A, VEGF-B, VEGF-C, VEGF-D, placental growth factor (PIGF), or a combination thereof. In some embodiments, the vascular maturation factor is, or comprises, a recombinant vascular maturation factor, a mutant vascular maturation factor, a fragment of the vascular maturation factor, or a combination thereof. The vascular maturation factor can be, or can comprise, platelet-derived growth factor (PDGF), angiopoietin-1 (Ang-1), or a combination thereof. The vascular maturation factor can be, or can comprise, PDGF subunit A, PDGF subunit B, PDGF subunit C, PDGF subunit D, or a combination thereof.

In some embodiments, non-leaking or minimally-leaking choroidal perfusion in the subject is increased after the composition is administered to the subject. Non-leaking or minimally-leaking choroidal revascularization in the subject can be promoted after the composition is administered to the subject. Macular flow voids can be reduced, hypoxia in the outer retina and retinal pigment epithelium (RPE) of the eye of the subject can be reduced, and/or ischemia in the outer retina and RPE of the eye of the subject can be reduced after the composition is administered to the subject. Non-leaking or minimally-leaking choroidal perfusion in the subject can increase after the composition is administered to the subject. Non-leaking or minimally-leaking retinal perfusion in the subject can increase after the composition is administered to the subject. Non-leaking or minimally-leaking retinal revascularization in the subject can be promoted after the composition is administered to the subject. Hypoxia in the retina of the eye of the subject can be reduced, and/or ischemia in the retina of the eye of the subject is reduced after the composition is administered to the subject. Non-leaking or minimally-leaking retinal perfusion in the subject can increase after the composition is administered to the subject. Exudation or neovascularization in the choroid or retina of the eye of the subject can be reduced after the composition is administered to the subject. Non-leaking or minimally-leaking choroidal perfusion and/or non-leaking and/or minimally-leaking retinal perfusion in the subject can increase by at least 5% after the composition is administered to the subject. New non-leaking or minimally-leaking blood vessels can form in the choroid and/or the retina of the eye of the subject after the composition is administered to the subject. Retinal edema, subretinal fluid, or both, in the subject can be reduced after the composition is administered to the subject. Leaky choroidal neovascularization in the subject can be mitigated after the composition is administered to the subject.

In some embodiments, the subject has a disease that is dry age-related macular degeneration (AMD) and/or geographic atrophy (GA), or a combination thereof. The composition, after being administered to the subject, can result in reversing, halting, or slowing of the progression of the disease in the subject. The reversing, halting, or slowing of the progression of the disease can be mediated by increased choroidal perfusion and/or non-leaking or minimally-leaking choroidal revascularization. Macular atrophy, geographic atrophy (GA), or both in the subject can be mitigated after the composition is administered to the subject.

In some embodiments, the subject has a disease that is wet age-related macular degeneration (AMD), choroidal neovascularization (CNV), polypoidal choroidal vasculopathy, degenerative (pathologic) myopia, or a combination thereof. The composition, after being administered to the subject, can result in reversing, halting, or slowing the progression of the disease in the subject. The reversing, halting, or slowing of the progression of the disease can be mediated by increased choroidal perfusion and/or non-leaking or minimally-leaking choroidal revascularization.

In some embodiments, the subject has a disease that is diabetic macular edema, macular edema from retinal vein occlusion, diabetic retinopathy, retinal vein occlusion, retinopathy of prematurity, retinal neovascularization, optic nerve neovascularization, familial exudative vitreoretinopathy, sickle cell disease, or a combination thereof. The composition, after being administered to the subject, can result in halting or slowing of the progression of the disease in the subject. The halting or slowing of the progression of the disease can be mediated by increased non-leaking or minimally-leaking retinal perfusion and/or non-leaking or minimally-leaking retinal revascularization.

In some embodiments, the subject has a disease that is radiation retinopathy, radiation optic neuropathy, or a combination thereof. The composition, after being administered to the subject, can result in reversing, halting, or slowing of the progression of the disease in the subject. The reversing, halting, or slowing of the progression of the disease can be mediated by increased non-leaking or minimally-leaking retinal perfusion, non-leaking or minimally-leaking retinal revascularization and/or non-leaking or minimally-leaking revascularization of the optic nerve of the eye of the subject. The subject may have received a radiation treatment for a disease that is an intraocular tumor a head tumor, a neck tumor, or a combination thereof, resulting in delayed onset of the disease. The administration of the composition to the subject occurs about 1-26 weeks after the subject receives the radiation treatment.

In some embodiments, the progression of the ocular disease in the subject is treated or slowed after the composition is administered to the subject. In some embodiments, a hypoxia inducible factor (HIF)-mediated blinding complication in the subject is mitigated after the composition is administered to the subject. The HIF-mediated visual loss complication can comprise choroidal neovascularization (CNV), retinal neovascularization, macular edema, or a combination thereof.

In some embodiments, the composition is for intravitreal administration. In some embodiments, the composition is for subretinal administration. In some embodiments, the composition is for administration to the suprachoroidal space of an eye of the subject. The composition can be for administration to the macular region of an eye of the subject. The composition can be for administration to one or more retinal or choroidal regions, of the eye of the subject, with reduced perfusion. The composition can be for administration to the subject about once every week to about once every year. The composition can be for administration to the subject over about one day to about 10 years.

In some embodiments, the formulation comprises about 0.001 mg/ml to about 200 mg/ml of the pro-angiogenic factor. The formulation cans comprise about 0.001 mg/ml to about 200 mg/ml of the vascular maturation factor.

Disclosed herein include embodiments of a kit comprising: a formulation comprising a pro-angiogenic factor and/or a vascular maturation factor; and a label indicating that the formulation is for increasing choroidal perfusion and/or retinal perfusion. Disclosed herein include embodiments of a kit comprising: a formulation comprising a pro-angiogenic factor and/or a vascular maturation factor; and a label indicating that the formulation is for treating an ocular disease associated with, or characterized by, choroidal hypoperfusion or retinal hypoperfusion. Disclosed herein include embodiments of a kit comprising: a formulation comprising an angiogenesis factor; and a label indicating that the formulation is for increasing choroidal perfusion and/or retinal perfusion. Disclosed herein include embodiments of a kit comprising: a formulation comprising an angiogenesis factor; and a label indicating that the formulation is for treating an ocular disease associated with, or characterized by, choroidal hypoperfusion and/or retinal hypoperfusion. The angiogenesis factor can be, or can comprise, a pro-angiogenic factor and/or a vascular maturation factor.

In some embodiments, the pro-angiogenic factor is, or comprises, a recombinant pro-angiogenic factor, a mutant pro-angiogenic factor, a fragment of the pro-angiogenic factor, or a combination thereof. The pro-angiogenic factor can be, or can comprise, vascular endothelial growth factor (VEGF), angiopoietin-2 (Ang-2), or a combination thereof. The VEGF can be, or can comprise, VEGF-A, VEGF-B, VEGF-C, VEGF-D, placental growth factor (PIGF), or a combination thereof. In some embodiments, the vascular maturation factor is, or comprises, a recombinant vascular maturation factor, a mutant vascular maturation factor, a fragment of the vascular maturation factor, or a combination thereof. The vascular maturation factor can be, or can comprise, platelet-derived growth factor (PDGF), angiopoietin-1 (Ang-1), or a combination thereof. The vascular maturation factor can be, or can comprise, PDGF subunit A, PDGF subunit B, PDGF subunit C, PDGF subunit D, or a combination thereof.

In some embodiments, non-leaking or minimally-leaking choroidal perfusion in the subject is increased after the formulation is administered to the subject. Non-leaking or minimally-leaking choroidal revascularization in the subject can be promoted after the formulation is administered to the subject. Macular flow voids can be reduced, hypoxia in the outer retina and retinal pigment epithelium (RPE) of the eye of the subject can be reduced, and/or ischemia in the outer retina and RPE of the eye of the subject can be reduced after the formulation is administered to the subject. Non-leaking or minimally-leaking choroidal perfusion in the subject can be increased after the formulation is administered to the subject.

In some embodiments, the label indicates the formulation is for treating a disease selected from a group comprising dry age-related macular degeneration (AMD) and/or geographic atrophy (GA), or a combination thereof. In some embodiments, the label indicates the formulation is for treating a disease selected from a group comprising wet age-related macular degeneration (AMD), choroidal neovascularization (CNV), polypoidal choroidal vasculopathy, degenerative (pathologic) myopia, or a combination thereof. In some embodiments, the label indicates the formation is for treating a disease selected from a group comprising diabetic macular edema, macular edema from retinal vein occlusion, diabetic retinopathy, retinal vein occlusion, retinopathy of prematurity, retinal neovascularization, optic nerve neovascularization, familial exudative vitreoretinopathy, sickle cell disease, or a combination thereof. Non-leaking or minimally-leaking retinal perfusion in the subject can increase after the formulation is administered to the subject. Non-leaking or minimally-leaking retinal revascularization in the subject can be promoted after the formulation is administered to the subject. Hypoxia in the retina of the eye of the subject can be reduced, and/or ischemia in the retina of the eye of the subject can be reduced after the formulation is administered to the subject. Non-leaking or minimally-leaking retinal perfusion in the subject can increase after the formulation is administered to the subject.

In some embodiments, the label indicates the formulation is for treating a disease that is radiation retinopathy, radiation optic neuropathy, or a combination thereof. The label can indicate the formulation is for treating the subject after the subject receives a radiation treatment for a disease that is an intraocular tumor, a head tumor, a neck tumor, or a combination thereof, resulting in delayed onset of the disease. The label can indicate the administration of the formulation (for example, any of the pharmaceutical formulations disclosed herein) to the subject can occur about 1-26 weeks after the subject receives the radiation treatment. In some embodiments, the label indicates the formulation is for treating a hypoxia inducible factor (HIF)-mediated blinding complication. The HIF-mediated visual loss complication comprises choroidal neovascularization (CNV), retinal neovascularization, macular edema, or a combination thereof.

In some embodiments, exudation or neovascularization in the choroid or retina of the eye of the subject is reduced after the formulation is administered to the subject. Non-leaking or minimally-leaking choroidal perfusion and/or non-leaking and/or minimally-leaking retinal perfusion in the subject can increase by at least 5% after the formulation is administered to the subject. New non-leaking or minimally-leaking blood vessels can be formed in the choroid and/or the retina of the eye of the subject after the formulation is administered to the subject. Retinal edema, subretinal fluid, or both, in the subject can be reduced after the formulation is administered to the subject. Leaky choroidal neovascularization in the subject can be mitigated after the formulation is administered to the subject. Macular atrophy, geographic atrophy (GA), or both in the subject can be mitigated after the formulation is administered to the subject.

In some embodiments the composition is for intravitreal administration. The composition can be formulated for subretinal administration. The composition can be formulated for administration to the suprachoroidal space of an eye of the subject. The composition can be formulated for administration to the macular region of an eye of the subject. The composition can be formulated for administration to one or more retinal or choroidal regions, of the eye of the subject, with reduced perfusion. The composition can be formulated for administration to the subject about once every week to about once every year. The composition can be formulated for administration to the subject over about 1 day to about 10 years.

In some embodiments, the formulation comprises about 0.001 mg/ml to about 10 mg/ml of the pro-angiogenic factor. The formulation can optionally comprise about 0.001 mg/ml to about 10 mg/ml of the vascular maturation factor.

Details of one or more implementations of the subject matter described in this specification are set forth in the accompanying drawings and the description below. Other features, aspects, and advantages will become apparent from the description, the drawings, and the claims. Neither this summary nor the following detailed description purports to define or limit the scope of the inventive subject matter.

### BRIEF DESCRIPTION OF THE DRAWINGS

FIG. 1A. is a Fundus photograph with geographic atrophy (GA) in right eye outlined. FIG. 1B is an OCT angiogram of a higher magnification view showing border of GA (blue) and adjacent regions or choriocapillaris hypo-perfusion outlined (red).
FIG. 2 is an OCT angiogram showing that CNV is surrounded by zone of choriocapillaris hypo-perfusion (outline).
FIG. 3 is an OCT angiogram (Zeiss Angioplex) from a patient 12 months after discontinuation of anti-VEGF injection in OD. There was a mature CNV underlying the fovea with surrounding choriocapillaris hypo-perfusion. Vision is excellent.
FIG. 4 is a schematic illustration showing an animal study protocol for oxygen-induced retinopathy of prematurity model.
FIG. 5 is a schematic illustration showing another animal study protocol for treatment of oxygen-induced retinopathy of prematurity model with revascularization factor.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

All patents, published patent applications, other publications, and sequences from GenBank, and other databases referred to herein are incorporated by reference in their entirety with respect to the related technology.

There are two blood supplies to the retina. The central retinal artery and its branches supply the inner half of the retina; the choroid supplies the outer half of the retina. The innermost layer of the choroid is the choriocapillaris, which supplies the retinal pigment epithelium and photoreceptors in the macula. The major causes of visual loss in the Western world, diabetic retinopathy and age-related macular degeneration, result in damage to the retinal capillaries and choriocapillaris, respectively. Damage to these capillary beds results in capillary non-perfusion and ischemia. The consequences of capillary ischemia, whether to the inner or outer retina, can be devastating to vision.

In the case of retinal capillary non-perfusion, resulting hypoxia induces secretion of vascular endothelial growth factor (VEGF), which promotes capillary leakage and macular edema, the most common cause of visual loss in diabetic retinopathy. Extensive capillary non-perfusion in diabetic retinopathy and increased levels of VEGF secretion promote development of retinal and optic nerve neovascularization, which can bleed into the vitreous cavity and/or cause retinal detachment. Anti-VEGF therapy (e.g. ranibizumab, bevacizumab, aflibercept) that is injected into the vitreous cavity multiple times per year inactivates VEGF and often improves visual function in these patients. Because the underlying ischemia (capillary non-perfusion) often persists, ongoing monitoring and frequent intraocular injection of anti-VEGF therapy remain necessary for these diabetic patients. A host of other retinovascular diseases similarly cause retinal capillary non-perfusion resulting in macular edema and/or neovascularization. These diseases include but are not limited to: retinal vein occlusion, retinopathy of prematurity, ocular ischemic syndrome, radiation retinopathy, radiation optic neuropathy, sickle cell retinopathy, and Eale's disease. Macular edema or neovascularization in these other retinovascular diseases can likewise be treated using anti-VEGF therapy.

Radiation retinopathy and optic neuropathy are observed months to years after irradiation of the eye or head and neck. Radiation retinopathy has clinical manifestations very similar to diabetic retinopathy: capillary closure, hemorrhages, cotton wool spots, macular edema, and neovascularization. Radiation optic neuropathy initially produces disc swelling, peripapillary hemorrhages and exudates. With time, this usually progresses to severe visual loss with optic atrophy. As with radiation retinopathy, the capillary endothelium is primarily damaged. The superficial optic nerve capillaries of retinal origin are particularly susceptible.

OCTA study of patients with radiation retinopathy reveals enlargement of the foveal avascular zone and damage to both the superficial and deep retinal capillary plexuses. OCTA demonstrates loss of radial peripapillary capillaries in patients with radiation optic neuropathy.

Treatment of radiation retinopathy is largely directed at exudative complications. As in therapy of diabetic macular edema, radiation related macular edema can respond to anti-VEGF therapy. For patients with visual loss from erosion of the perifoveal capillary bed, there is no effective therapy to restore capillary integrity. Similarly, there are no established effective therapies for treatment of radiation optic neuropathy secondary to capillary closure.

The outer retinal blood supply, the choriocapillaris, is also subject to damage and development of reduced capillary perfusion. In age-related macular degeneration (AMD), both histologic analysis and optical coherence tomography angiography (OCTA) studies reveal reduced choriocapillaris perfusion. Resulting hypoxia can promote VEGF secretion and development of choroidal neovascularization ("wet AMD"). As in the case of hypoxia-driven VEGF secretion in diabetic retinopathy, choroidal neovascularization is managed with repeated intravitreal injection of anti-VEGF therapy to suppress the neovascularization.

Therefore, in both diabetic retinopathy (and similar diseases causing retinal capillary non-perfusion) and age-related macular degeneration, anti-VEGF therapy is used to treat the consequences of retinal ischemia induced by capillary non-perfusion. While anti-VEGF therapy is effective in these conditions, it does not treat the underlying ischemia resulting from capillary drop-out. Hence, repeated intravitreal injections of anti-VEGF agents are needed to manage ongoing retinal ischemia.

An alternative therapy would be to correct the underlying capillary drop out in the retina and/or the choriocapillaris by revascularizing these damaged capillary beds. In other words, rather than relying on a temporary approach that suppresses the effects of VEGF, i.e. anti-angiogenic therapy, a preferred approach might be to actually promote revascularization (angiogenesis) of the retina and/or choriocapillaris using pro-angiogenic factor(s). Using this approach, the regions of capillary drop-out could be revascularized, which would reduce levels of VEGF and their visually disabling consequences, namely, macular exudation and neovascularization. Because macular exudation is so deleterious to vision, any revascularization of the retina or choroid capillary beds would have to be minimally- or non-leaking (i.e., mature) to prevent visual loss.

Delivery of pro-angiogenic and vascular maturation growth factors to the eye is very counter-intuitive. As noted above, current therapies to treat neovascular diseases such as AMD, degenerative myopia, and diabetic retinopathy use anti-VEGF (anti-angiogenic) directed therapies. Our invention seeks to combat neovascularization not by blocking it; instead, by using pro-angiogenic factor(s) to revascularize the retina or choroid, the hypoxic stimulus for VEGF secretion is removed. Thus, anti-VEGF therapy is no longer needed, or at worst, the need for such therapy is significantly diminished.

In advanced AMD, geographic atrophy (GA) can develop in the macula and cause severe central visual loss. With GA, there is progressive loss of RPE, photoreceptors, and choriocapillaris in the macula. Studies have shown that the growing margin of GA displays choriocapillaris perfusion defects and that these perfusion defects can pre-date loss of RPE or overlying photoreceptors. Given the presence of choriocapillaris hypo-perfusion we observe in patients with GA, choroidal revascularization in the macular region, particularly at the growing edge of GA may also mitigate progression of this currently untreatable form of advanced AMD.

There is clinical support for revascularization as a means to prevent GA progression. There are AMD patients who develop both choroidal neovascularization and GA. If the choroidal neovascularization in these patients is mature (minimally- or non-leaking), the overlying retina appears "immune" to developing GA. OCT angiography has shown that patients with mature, sub-RPE choroidal neovascularization (CNV) underneath the macula develop GA eccentric to the retina overlying the neovascularization but not over the CNV itself. In these cases, mature CNV has grown in response to and takes the place of a hypo-perfused choriocapillaris. This suggests that the mature (minimally- or non-leaking) CNV maintains the viability of the overlying macular tissue and protects it from developing GA. Since this is a rather uncommon natural phenomenon and since most GA patients do not develop CNV, the phenomenon of CNV in wet AMD protecting against GA is supportive of therapeutic revascularization, but cannot reliably substitute for it. Rather than hoping a CNV is mature and provides sufficient metabolic support to the overlying macula to prevent progression of GA, revascularization with pro-angiogenic therapy predictably can achieve sufficient choriocapillaris perfusion to prevent progression of GA.

While the role of choriocapillaris non-perfusion in advanced AMD is described above, choriocapillaris revascularization can also mitigate progression of degenerative myopia and polypoidal choroidal vasculopathy, both of which are associated with choroidal hypo-perfusion, choroidal neovascularization, and geographic atrophy.

### Age-Related Macular Degeneration and Optical Coherence Tomography

For the past decade, cases of wet AMD have been managed using serial intravitreal injections of anti-vascular endothelial growth factor (anti-VEGF) agents. Following diagnosis of wet AMD based on clinical exam and supplemented typically by imaging using optical coherence tomography (OCT) and/or fluorescein angiography (FA), a series of anti-VEGF injections is initiated. Ordinarily, a loading series of injections (ordinarily three injections given over two months) is administered, after which patients are closely followed using clinical exam and OCT to determine when further injections are indicated. Some surgeons manage patients using monthly injections regardless of clinical and OCT findings; others inject on a pro re nata (PRN) or "treat and extend" regimen. These anti-VEGF injections can be very effective at stabilizing and often, improving visual acuity in patients with wet AMD.

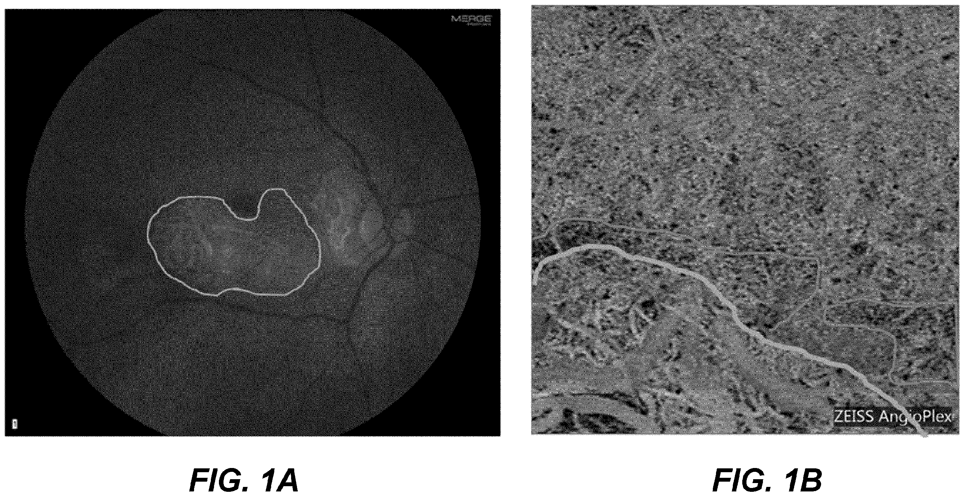

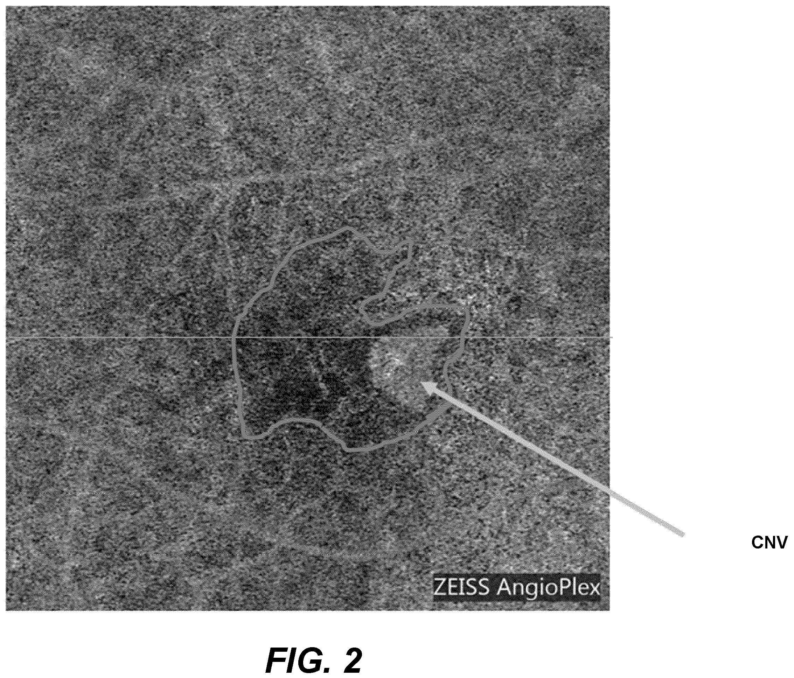

Recent development of OCT angiography (OCT-A), has, for the first time, enabled visualization of the choriocapillaris (CC), the blood supply to the macular RPE and overlying photoreceptors. It has been demonstrated CC perfusion defects surround regions of geographic atrophy in dry AMD (FIGS. 1A and 1B); similar regions of CC hypo-perfusion are observed at the margins of CNV in wet AMD (FIG. 2). These zones of CC hypo-perfusion surrounding GA and CNV are consistent with post-mortem immunohistochemical studies of AMD that similarly demonstrate CC hypo-perfusion in dry and wet AMD. The presence of CC perfusion defects in incipient regions of GA and around the leading edge of CNV suggest that hypoxia may play a role in progression of AMD.

The primary focus of treating wet AMD has been directed at neutralizing VEGF with monoclonal antibodies, monoclonal antibody fragments, and recombinant fusion proteins of VEGF binding portions of VEGF receptors ("VEGF trap"). Ocular therapies directed at the stimulus for VEGF secretion have not been implemented clinically.

The primary stimulus for VEGF secretion is hypoxia inducible factor (HIF). HIFs are a group of transcription factors secreted in response to hypoxia. Once activated, HIF induces transcription of multiple pro-angiogenic factors including, VEGF, VEGFR-1, PDGF-B, SDF-1, Ang-2, and EPO. An alternative therapeutic target to VEGF as a means to inhibit ocular neovascularization would be to inhibit HIF. Because HIF is exquisitely sensitive to oxygen concentration, and because there is CC hypo-perfusion in both dry and wet AMD, increasing outer retinal oxygen levels could suppress HIF and thereby inhibit neovascularization.

Means to selectively increase local oxygen levels to the outer retina are not available. Systemic oxygen administration via nasal cannula or by hyperbaric oxygen chamber has been demonstrated to reduce VEGF mediated effects in diabetic macular edema and macular edema secondary to retinal vein occlusion. Despite anecdotal efficacy, systemic administration of oxygen to treat a locally hypoxic retina is cumbersome.

### Increasing Oxygen Level

An alternative means to locally increase oxygen levels in the retina could mitigate retinal hypoxia and reduce HIF mediated blinding complications such as CNV, retinal NV, and macular edema. One such method would be to revascularize the choroid by treating the eye locally with pro-angiogenic growth factors. A revascularized choroid would supply required oxygen to the outer retina, which would reduce secretion of VEGF and other pro-angiogenic growth factors.

Revascularization in the heart can be used to treat ischemic cardiac disease. A variety of growth factors can be injected into the heart to promote neovascularization. These include: vascular endothelial growth factor (VEGF), fibroblast growth factor (FGF), platelet-derived growth factor (PDGF), stromal-derived factor 1-a (SDF1-α), insulin growth factor-1 (IGF-1), hepatocyte growth factor (HGF), angiopoietin-2 (Ang-2), and angiopoietin-1 (Ang-1). While VEGF is effective at promoting neovascularization, the new vessels are immature and hyper-permeable, similar to the vessels we observe in pathologic choroidal neovascularization with AMD. Providing additional growth factor(s), such as Ang-1 and PDGF, which promote maturation of neovascularization can promote formation of more mature neovascular networks. PDGF recruits pericytes and smooth muscle cells to stabilize immature neovascularization. Ang-1 likewise stabilizes vascular endothelial-pericyte interactions. These growth factor "cocktails" can be administered at once or delivered sequentially using a variety of sustained release formulations. VEGF in fibrin gel and PDGF in a heparin-based coacervate can be used to effect sequential release of these growth factors (e.g., in a rat model of acute myocardial infarction). Such polymer release systems can provide spatio-temporal control over growth factor release; they also enable release over a prolonged period of time to maintain a mature vascular network. Sequential administration of pro-angiogenic growth factors (VEGF, Ang-2) followed by administration of vascular maturation growth factors (PDGF, Ang-1) can result in development of a mature neovascular network in an animal model.

Since newly formed choroidal vessels grow toward the region of concentrated growth factors, the revascularization formulation could be delivered to the suprachoroidal space subjacent to the macular region. There, the pro-angiogenic and vascular maturation factors would diffuse toward the choroid and promote revascularization.

The pro-angiogenic and vascular maturation factors could also be injected intraocularly in the vitreous and/or underneath the retina. Revascularization could be monitored non-invasively using optical coherence tomography angiography (OCT-A). If revascularization is inadequate, additional growth factors could be injected. If revascularization is excessive, anti-VEGF therapy could be instituted. Further, improved blood supply to the outer retina in cases of wet AMD would reduce levels of secreted VEGF by retinal pigment epithelial cells. This would have the same effects of injecting intravitreal anti VEGF agents; namely, reduction in retinal edema and subretinal fluid. This reduction in retinal exudation caused by revascularization of the choroid would be measurable using conventional OCT imaging. In the case where immature neovascularization was imaged by OCT-A, but the vessels remained leaky with associated macular edema and subretinal fluid, additional vascular maturation factors (e.g., PDGF, Ang-1) could be administered. Effects of vascular maturation on exudation could be readily monitored using OCT. The combination of OCT and OCT-A enable detection of both the extent and maturity of revascularized choroidal networks following growth factor therapy.

Disclosed herein include embodiments of a method for increasing choroidal perfusion in a subject in need thereof. In some embodiments, the method comprises: administering a formulation comprising a therapeutically effective amount of a pro-angiogenic factor and/or a vascular maturation factor to the subject in need of increased choroidal perfusion. In some embodiments, the method results in increasing non-leaking or minimally-leaking choroidal perfusion in the subject. Disclosed herein include embodiments of a method for promoting non-leaking or minimally-leaking choroidal revascularization in a subject in need thereof. In some embodiments, the method comprises: administering a formulation comprising a therapeutically effective amount of a pro-angiogenic factor and/or a vascular maturation factor to the subject in need of non-leaking or minimally-leaking choroidal revascularization. In some embodiments, the method results in promoting non-leaking or minimally-leaking choroidal revascularization in the subject. Disclosed herein include embodiments of a method for increasing choroidal perfusion in a subject in need thereof. In some embodiments, the method comprises: administering a formulation comprising a therapeutically effective amount of an angiogenesis or angiogenic factor or agent to the subject in need of increased choroidal perfusion. In some embodiments, the method results in increasing non-leaking or minimally-leaking choroidal perfusion in the subject. Disclosed herein include embodiments of a method for promoting non-leaking or minimally-leaking choroidal revascularization in a subject in need thereof. In some embodiments, the method comprises: administering a formulation comprising a therapeutically effective amount of an angiogenesis or angiogenic factor or agent to the subject in need of non-leaking or minimally-leaking choroidal revascularization. In some embodiments, the method results in promoting non-leaking or minimally-leaking choroidal revascularization in the subject. The angiogenesis or angiogenic factor or agent can comprise, or can be, a pro-angiogenic factor and/or a vascular maturation factor. Disclosed herein include embodiments of a method for increasing choroidal perfusion in a subject in need thereof. In some embodiments, the method comprises: causing a level of a pro-angiogenic factor in the choroid of an eye of the subject to increase; and administering a therapeutically effective amount of a vascular maturation factor to the subject. In some embodiments, the method results in increasing non-leaking or minimally-leaking choroidal perfusion in the subject. Disclosed herein include embodiments of a method for promoting non-leaking or minimally-leaking choroidal revascularization in a subject in need thereof. In some embodiments, the method, comprises: causing a level of a pro-angiogenic factor in the choroid of an eye of the subject to increase; and administering a therapeutically effective amount of a vascular maturation factor to the subject. In some embodiments, the method results in promoting non-leaking or minimally-leaking choroidal revascularization in the subject.

Disclosed herein include embodiments of a method for increasing retinal perfusion in a subject in need thereof. In some embodiments, the method comprises: administering a formulation comprising a therapeutically effective amount of a pro-angiogenic factor and/or a vascular maturation factor, to the subject in need of increased retinal perfusion. In some embodiments, the method results in increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: administering a formulation comprising a therapeutically effective amount of a pro-angiogenic factor and/or a vascular maturation factor to the subject in need of non-leaking or minimally-leaking retinal revascularization. In some embodiments, the method results in promoting non-leaking or minimally-leaking retinal revascularization in the subject. Disclosed herein include embodiments of a method for increasing retinal perfusion in a subject in need thereof. In some embodiments, the method comprises: administering a formulation comprising a therapeutically effective amount of an angiogenesis or angiogenic factor or agent to the subject in need of increased retinal perfusion. In some embodiments, the method results in increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: administering a formulation comprising a therapeutically effective amount of an angiogenesis or angiogenic factor or agent to the subject in need of non-leaking or minimally-leaking retinal revascularization. In some embodiments, the method results in promoting non-leaking or minimally-leaking retinal revascularization in the subject. The angiogenesis or angiogenic factor or agent can comprise, or can be, a pro-angiogenic factor and/or a vascular maturation factor. Disclosed herein include embodiments of a method for increasing retinal perfusion in a subject in need thereof. In some embodiments, the method comprises: causing a level of a pro-angiogenic factor in the retina of an eye of the subject in need of increased retinal perfusion to increase; and administering a therapeutically effective amount of a vascular maturation factor to the subject. In some embodiments, the method results in increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: causing a level of a pro-angiogenic factor in the retina of an eye of the subject in need of non-leaking or minimally-leaking retinal revascularization to increase; and administering a therapeutically effective amount of a vascular maturation factor to the subject. In some embodiments, the method results in promoting non-leaking or minimally-leaking retinal revascularization in the subject.

Disclosed herein include embodiments of a method for treating an ocular disease in a subject in need thereof. In some embodiments, the method comprises: administering a formulation to the subject, wherein the formulation comprises a therapeutically effective amount of a pro-angiogenic factor and/or a vascular maturation factor. In some embodiments, the method results in treating or slowing the progression of the ocular disease in the subject. Disclosed herein include embodiments of a method for treating an ocular disease in a subject in need thereof. In some embodiments, the method comprises: causing a level of a pro-angiogenic factor in the choroid or retina of an eye of the subject to increase; and administering a therapeutically effective amount of a vascular maturation factor to the subject, treating or slowing the progression of the ocular disease in the subject.

Disclosed herein include embodiments of a composition comprising a pro-angiogenic factor and/or a vascular maturation factor for use in increasing non-leaking or minimally-leaking choroidal perfusion or non-leaking or minimally-leaking retinal perfusion in a subject in need thereof. Disclosed herein include embodiments of a composition comprising a pro-angiogenic factor and/or a vascular maturation factor for use in the treatment of an ocular disease associated with, or characterized by, choroidal hypoperfusion or retinal hypoperfusion in a subject in need thereof. Disclosed herein include embodiments of a composition comprising an angiogenesis or angiogenic factor or agent for use in increasing non-leaking or minimally-leaking choroidal perfusion or non-leaking or minimally-leaking retinal perfusion in a subject in need thereof. Disclosed herein include embodiments of a composition comprising an angiogenesis for use in the treatment of an ocular disease associated with, or characterized by, choroidal hypoperfusion or retinal hypoperfusion in a subject in need thereof. The angiogenesis or angiogenic factor or agent can be, or can comprise, a pro-angiogenic factor and/or a vascular maturation factor.

Disclosed herein include embodiments of a composition comprising a pro-angiogenic factor and/or a vascular maturation factor for use in increasing non-leaking or minimally-leaking choroidal perfusion or non-leaking or minimally-leaking retinal perfusion in a subject in need thereof. Disclosed herein include embodiments of a composition comprising a pro-angiogenic factor and/or a vascular maturation factor for use in the treatment of an ocular disease associated with, or characterized by, choroidal hypoperfusion or retinal hypoperfusion in a subject in need thereof. Disclosed herein include embodiments of a composition comprising an angiogenesis or angiogenic factor or agent for use in increasing non-leaking or minimally-leaking choroidal perfusion or non-leaking or minimally-leaking retinal perfusion in a subject in need thereof. Disclosed herein include embodiments of a composition comprising an angiogenesis for use in the treatment of an ocular disease associated with, or characterized by, choroidal hypoperfusion or retinal hypoperfusion in a subject in need thereof.

### Increasing Non-Leaking or Minimally-Leaking Choroidal Perfusion

Disclosed herein include embodiments of a method for increasing choroidal perfusion in a subject in need thereof. In some embodiments, the method comprises: administering a formulation comprising a therapeutically effective amount of a pro-angiogenic factor and/or a vascular maturation factor to the subject in need of increased choroidal perfusion, thereby increasing non-leaking or minimally-leaking choroidal perfusion in the subject. Disclosed herein include embodiments of a method for promoting non-leaking or minimally-leaking choroidal revascularization in a subject in need thereof. In some embodiments, the method comprises: administering a formulation comprising a therapeutically effective amount of a pro-angiogenic factor and/or a vascular maturation factor to the subject in need of non-leaking or minimally-leaking choroidal revascularization, thereby promoting non-leaking or minimally-leaking choroidal revascularization in the subject.

Disclosed herein include embodiments of a method for increasing choroidal perfusion in a subject in need thereof. In some embodiments, the method comprises: administering a formulation comprising a therapeutically effective amount of an angiogenesis or angiogenic factor or agent to the subject in need of increased choroidal perfusion, thereby increasing non-leaking or minimally-leaking choroidal perfusion in the subject. Disclosed herein include embodiments of a method for promoting non-leaking or minimally-leaking choroidal revascularization in a subject in need thereof. In some embodiments, the method comprises: administering a formulation comprising a therapeutically effective amount of an angiogenesis or angiogenic factor or agent to the subject in need of non-leaking or minimally-leaking choroidal revascularization, thereby promoting non-leaking or minimally-leaking choroidal revascularization in the subject. The angiogenesis or angiogenic factor or agent can comprise, or can be, a pro-angiogenic factor and/or a vascular maturation factor.

Disclosed herein include embodiments of a method for increasing choroidal perfusion in a subject in need thereof. In some embodiments, the method comprises: causing a level of a pro-angiogenic factor in the choroid of an eye of the subject to increase; and administering a therapeutically effective amount of a vascular maturation factor to the subject, thereby increasing non-leaking or minimally-leaking choroidal perfusion in the subject. Disclosed herein include embodiments of a method for promoting non-leaking or minimally-leaking choroidal revascularization in a subject in need thereof. In some embodiments, the method, comprises: causing a level of a pro-angiogenic factor in the choroid of an eye of the subject to increase; and administering a therapeutically effective amount of a vascular maturation factor to the subject, thereby promoting non-leaking or minimally-leaking choroidal revascularization in the subject. Causing the level of the pro-angiogenic factor in the choroid of the eye of the subject to increase can comprise discontinuing or reducing the administration frequency of an antagonist of a second pro-angiogenic factor. The pro-angiogenic factor and the second pro-angiogenic factor can be identical, or different. The pro-angiogenic factor and the second pro-angiogenic factor can be different. The antagonist of the second pro-angiogenic factor can comprise an antibody targeting the second pro-angiogenic factor, or a fragment thereof. Causing the level of the second pro-angiogenic factor in the choroid of the eye of the subject to increase can comprise administering a therapeutically effective amount of the second pro-angiogenic factor to the subj ect.

In some embodiments, thereby macular flow voids are reduced, hypoxia in the outer retina and retinal pigment epithelium (RPE) of the eye of the subject is reduced, and/or ischemia in the outer retina and RPE of the eye of the subject is reduced. In some embodiments, choroidal perfusion is thereby increased in the subject.

In some embodiments, the method comprises: determining an extent of choroidal perfusion or non-leaking or minimally-leaking choroidal revascularization in the subject to be inadequate; and continue administering the formulation comprising the effective amount of the pro-angiogenic factor and/or the vascular maturation factor to the subject. The determining can comprise performing an ocular examination or sequential ocular examinations. The sequential ocular examinations can comprise visual acuity assessment, a fundus auto-fluorescence (FAF) examination, an optical coherence tomography (OCT) examination, an optical coherence tomography angiography (OCT-A) examination, a fluorescein angiography (FA) examination, an indocyanine green (ICG) angiography examination, or a combination thereof.

In some embodiments, the method comprises, prior to the administering, determining whether the subject is in need of increased choroidal perfusion or non-leaking or minimally-leaking choroidal revascularization with an ocular examination. In some embodiments, the subject has a disease selected from a group comprising dry age-related macular degeneration (AMD) and/or geographic atrophy (GA), or a combination thereof. The method can thereby result in reversing, halting, or slowing the progression of the disease. The reversing, halting or slowing of the progression of the disease can be mediated by increased choroidal perfusion and/or non-leaking or minimally-leaking choroidal revascularization.

In some embodiments, the subject has a disease selected from a group comprising wet age-related macular degeneration (AMD), choroidal neovascularization (CNV), polypoidal choroidal vasculopathy, degenerative (pathologic) myopia, giant cell arteritis, or a combination thereof. The method can thereby result in reversing, halting, or slowing the progression of the disease. The reversing, halting, or slowing of the progression of the disease can be mediated by increased choroidal perfusion and/or non-leaking or minimally-leaking choroidal revascularization. In some embodiments, the method comprises administering a therapeutically effective amount of an antagonist of a second pro-angiogenic factor that reduces vascular leakage while non-leaking or minimally-leaking choroidal neovascularization develops.

### Increasing Retinal Perfusion

Disclosed herein include embodiments of a method for increasing retinal perfusion in a subject in need thereof. In some embodiments, the method comprises: administering a formulation comprising a therapeutically effective amount of a pro-angiogenic factor and/or a vascular maturation factor to the subject in need of increased retinal perfusion, thereby increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: administering a formulation comprising a therapeutically effective amount of a pro-angiogenic factor and/or a vascular maturation factor to the subject in need of non-leaking or minimally-leaking retinal revascularization, thereby promoting non-leaking or minimally-leaking retinal revascularization in the subject.

Disclosed herein include embodiments of a method for increasing retinal perfusion in a subject in need thereof. In some embodiments, the method comprises: administering a formulation comprising a therapeutically effective amount of an angiogenesis or angiogenic factor or agent to the subject in need of increased retinal perfusion, thereby increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: administering a formulation comprising a therapeutically effective amount of an angiogenesis or angiogenic factor or agent to the subject in need of non-leaking or minimally-leaking retinal revascularization, thereby promoting non-leaking or minimally-leaking retinal revascularization in the subject. The angiogenesis or angiogenic factor or agent can comprise, or can be, a pro-angiogenic factor and/or a vascular maturation factor.

Disclosed herein include embodiments of a method for increasing retinal perfusion in a subject in need thereof. In some embodiments, the method comprises: causing a level of a pro-angiogenic factor in the retina of an eye of the subject in need of increased retinal perfusion to increase; and administering a therapeutically effective amount of a vascular maturation factor to the subject, thereby increasing non-leaking or minimally-leaking retinal perfusion in the subject. Disclosed herein include embodiments of a method for promoting non-leaking or minimally-leaking retinal revascularization in a subject in need thereof. In some embodiments, the method comprises: causing a level of a pro-angiogenic factor in the retina of an eye of the subject in need of non-leaking or minimally-leaking retinal revascularization to increase; and administering a therapeutically effective amount of a vascular maturation factor to the subject, thereby promoting non-leaking or minimally-leaking retinal revascularization in the subject. In some embodiments, causing the level of the pro-angiogenic factor in the retina of the eye of the subject to increase comprises discontinuing or reducing the administration frequency of an antagonist of a second pro-angiogenic factor. The pro-angiogenic factor and the second pro-angiogenic factor can be identical, or different. The antagonist of the second pro-angiogenic factor can comprise an antibody targeting the second pro-angiogenic factor, or a fragment thereof. In some embodiments, causing the level of the pro-angiogenic factor in the retina of the eye of the subject to increase comprises administering a therapeutically effective amount of the pro-angiogenic factor to the subject.

In some embodiments, thereby hypoxia in the retina of the eye of the subject is reduced, and/or ischemia in the retina of the eye of the subject is reduced. In some embodiments, the method thereby results in increasing non-leaking or minimally-leaking retinal perfusion in the subj ect.

In some embodiments, the method comprises: determining an extent of retinal perfusion or non-leaking or minimally-leaking retinal revascularization in the subject to be inadequate; and continue administering the formulation to the subject. The determining can comprise performing an ocular examination or sequential ocular examinations. The ocular examination or the sequential ocular examinations can comprise visual acuity assessment, an optical coherence tomography (OCT) examination, an optical coherence tomography angiography (OCT-A) examination, a fluorescein angiography (FA) examination, an indocyanine green (ICG) angiography examination, or a combination thereof.

In some embodiments, the method comprises, prior to the administering, determining the subject is in need of increased non-leaking or minimally-leaking retinal perfusion or non-leaking or minimally-leaking retinal revascularization with an ocular examination. In some embodiments, the subject has a disease selected from a group comprising diabetic macular edema, macular edema from retinal vein occlusion, diabetic retinopathy, retinal vein occlusion, retinopathy of prematurity, retinal neovascularization in diabetes, optic nerve neovascularization in diabetes, ocular ischemic syndrome, familial exudative vitreoretinopathy, sickle cell disease, or a combination thereof. The method can thereby result in halting or slowing the progression of the disease. The halting or slowing of the progression of the disease can be mediated by increased non-leaking or minimally-leaking retinal perfusion and/or non-leaking or minimally-leaking retinal revascularization. The method can comprise administering a therapeutically effective amount of an antagonist of a second pro-angiogenic factor.

In some embodiments, the subject has a disease selected from a group comprising radiation retinopathy, radiation optic neuropathy, or a combination thereof. The method can thereby result in reversing, halting, or slowing the progression of the disease. The reversing, halting or slowing of the progression of the disease can be mediated by increased non-leaking or minimally-leaking retinal perfusion, non-leaking or minimally-leaking retinal revascularization and/or non-leaking or minimally-leaking revascularization of the optic nerve of the eye of the subject. The subject can have received a radiation treatment for a disease selected from a group comprising an intraocular tumor, a head tumor, a neck tumor, or a combination thereof, resulting in delayed onset of radiation retinopathy and/or radiation optic neuropathy. The administering can comprise administering the formulation comprising the effective amount of the pro-angiogenic factor and/or the vascular maturation factor about 1-26 weeks after the subject receives radiation treatment.

### Treating Ocular Diseases

Disclosed herein include embodiments of a method for treating an ocular disease in a subject in need thereof. In some embodiments, the method comprises: administering a formulation to the subject, wherein the formulation comprises a therapeutically effective amount of a pro-angiogenic factor and/or a vascular maturation factor thereby treating or slowing the progression of the ocular disease in the subject. Disclosed herein include embodiments of a method for treating an ocular disease in a subject in need thereof. In some embodiments, the method comprises: causing a level of a pro-angiogenic factor in the choroid or retina of an eye of the subject to increase; and administering a therapeutically effective amount of a vascular maturation factor to the subject, treating or slowing the progression of the ocular disease in the subject. In some embodiments, causing the level of the pro-angiogenic factor in the choroid of the eye of the subject to increase comprises discontinuing or reducing the administration frequency of an antagonist of a second pro-angiogenic factor. The pro-angiogenic factor and the second pro-angiogenic factor can be identical. The antagonist of the second pro-angiogenic factor can comprise an antibody targeting the second pro-angiogenic factor, or a fragment thereof. In some embodiments, causing the level of the pro-angiogenic factor in the choroid of the eye of the subject to increase comprises administering a therapeutically effective amount of the pro-angiogenic factor to the subject.

In some embodiments, the ocular disease is wet age-related macular degeneration (AMD), polypoidal choroidal vasculopathy (PCV), degenerate (pathologic) myopia, or a combination thereof. In some embodiments, the ocular disease is associated with, or characterized by, choroidal hypoperfusion, choroidal neovascularization (CNV), macular atrophy, or a combination thereof. In some embodiments, the ocular disease is dry age-related macular degeneration (AMD), diabetic macular edema, macular edema from retinal vein occlusion, diabetic retinopathy, retinal vein occlusion, retinopathy of prematurity, retinal neovascularization in diabetes, optic nerve neovascularization in diabetes, familial exudative vitreoretinopathy, radiation retinopathy, radiation optic neuropathy, sickle cell disease, or a combination thereof. In some embodiments, the ocular disease is associated with, or characterized by, retinal hypoperfusion, retinal ischemia, optic nerve ischemia, or a combination thereof.

In some embodiments, the method comprises determining the severity of the ocular disease in the subject or the rate of progression of the ocular disease in the subject. In some embodiments, the method comprises identifying the subject as needing increased choroidal perfusion, as needing non-leaking or minimally-leaking choroidal revascularization. In some embodiments, the method comprises identifying the subject as a patient requiring increased retinal perfusion, non-leaking or minimally-leaking retinal revascularization, suffering from the ocular disease, or a combination thereof. In some embodiments, the subject is known to need increased choroidal perfusion, non-leaking or minimally-leaking choroidal revascularization, increased retinal perfusion, non-leaking or minimally-leaking retinal revascularization. In some embodiments, the subject is known to have the ocular disease.

### Factors

In some embodiments, the pro-angiogenic factor is, or comprises, a recombinant pro-angiogenic factor, a mutant pro-angiogenic factor, a fragment of the pro-angiogenic factor, or a combination thereof. The pro-angiogenic factor can be, or can comprise, vascular endothelial growth factor (VEGF), angiopoietin-2 (Ang-2), or a combination thereof. The VEGF can be, or can comprise, VEGF-A, VEGF-B, VEGF-C, VEGF-D, placental growth factor (PIGF), or a combination thereof. In some embodiments, the vascular maturation factor is, or comprises, a recombinant vascular maturation factor, a mutant vascular maturation factor, a fragment of the vascular maturation factor, or a combination thereof. The vascular maturation factor can be, or can comprise, platelet-derived growth factor (PDGF), angiopoietin-1 (Ang-1), or a combination thereof. The vascular maturation factor can be, or can comprise, PDGF subunit A, PDGF subunit B, PDGF subunit C, PDGF subunit D, or a combination thereof.

In some embodiments, one or more factors are administered to a subject to increase choroidal perfusion, to promote non-leaking or minimally-leaking choroidal revascularization, to increase retinal perfusion, to promote non-leaking or minimally-leaking retinal revascularization, and/or to treat an ocular disease in a subject in need thereof. The factors can be, or can comprise, adrenomedullin (AM), angiopoietin (Ang, ANGPT) (e.g., ANG-1, ANG-2, ANG-3, and ANG-4), Angiopoietin-related protein (ANGPTL) (e.g., ANGPTL1, ANGPTL2, ANGPTL3, ANGPTL4, ANGPTL5, ANGPTL6, ANGPTL7, and ANGPTL8), autocrine motility factor (AMF) (also known as glucose-6-phosphate isomerase (GPI), phosphoglucose isomerase/phosphoglucoisomerase (PGI) or phosphohexose isomerase (PHI)), bone morphogenetic protein (BMP) (e.g., BMP1, BMP2, BMP3, BMP4, BMP5, BMP6, BMP7, BMP8a, BMP8b, BMP10, BMP11, and BMP15), ciliary neurotrophic factor family protein (e.g., ciliary neurotrophic factor (CNTF), leukemia inhibitory factor (LIF), and interleukin-6 (IL-6)), colony-stimulating factor (e.g., macrophage colony-stimulating factor (M-CSF)/CSF1, granulocyte colony-stimulating factor (G-CSF)/CSF2, and granulocyte macrophage colony-stimulating factor (GM-CSF)/CSF3), epidermal growth factor (EGF), EGF-family protein (e.g., EGF, heparin-binding EGF-like growth factor (HB-EGF), transforming growth factor-a (TGF-α), amphiregulin (AR), epiregulin (EPR), epigen, betacellulin (BTC), neuregulin-1 (NRG1), neuregulin-2 (NRG2), neuregulin-3 (NRG3), and neuregulin-4 (NRG4)), ephrin (e.g., ephrin A1, ephrin A2, ephrin A3, ephrin A4, ephrin A5, ephrin B 1, ephrin B2, and ephrin B3), erythropoietin (EPO), fibroblast growth factor (FGF) (e.g., FGF1, FGF2, FGF3, FGF4, FGF5, FGF6, FGF7, FGF8, FGF9, FGF10, FGF11, FGF12, FGF13, FGF14, FGF15, FGF16, FGF17, FGF18, FGF19, FGF20, FGF21, FGF22, and FGF23), foetal bovine somatotrophin (FBS), GDNF family of ligand (e.g., glial cell line-derived neurotrophic factor (GDNF), neurturin, persephin, and artemin), growth differentiation factor-9 (GDF9), hepatocyte growth factor (HGF), hepatoma-derived growth factor (HDGF), insulin, insulin-like growth factors (e.g., insulin-like growth factor-1 (IGF-1), and insulin-like growth factor-2 (IGF-2)), interleukin (IL) (e.g., IL-1, IL-2, IL-3, IL-4, IL-5, IL-6, and IL-7), keratinocyte growth factor (KGF), migration-stimulating factor (MSF), macrophage-stimulating protein (MSP) (also known as hepatocyte growth factor-like protein (HGFLP)), myostatin (GDF-8), neuregulin (NRG) (e.g., NRG1, NRG2, NRG3, and NRG4), neurotrophin (e.g., brain-derived neurotrophic factor (BDNF), nerve growth factor (NGF), neurotrophin-3 (NT-3), and neurotrophin-4 (NT-4)), placental growth factor (PGF), platelet-derived growth factor (PDGF), renalase (RNLS), T-cell growth factor (TCGF), thrombopoietin (TPO), transforming growth factor (e.g., transforming growth factor alpha (TGF-α), and transforming growth factor beta (TGF-β)), tumor necrosis factor-alpha (TNF-α), vascular endothelial growth factor (VEGF) (e.g., VEGF-A, VEGF-B, VEGF-C, VEGF-D, PIGF), Wnt Signaling Pathway (WNT) protein (e.g., WNT1, WNT2, WNT2B, WNT3, WNT3A, WNT4, WNT5A, WNT5B, WNT6, WNT7A, WNT7B, WNT8A, WNT8B, WNT9A, WNT9B, WNT 10A, WNT10B, WNT 11, WNT 16) or a combination thereof.

In some embodiments, the one or more factors administered can be an angiogenesis or angiogenic factor or agent. The angiogenesis or angiogenic factor or agent can be, or can comprise, fibroblast growth factor (FGF), vascular endothelial growth factor (VEGF), VEGF receptor (VEGFR) (e.g., VEGFR-1, VEGFR-2, and VEGFR-3), neuropilin 1 (NRP-1), angiopoietin 1 (Ang1), angiopoietin 2 (Ang2), platelet-derived growth factor (PDGF) (e.g., BB-homodimer), PDGF receptor (PDGFR), transforming growth factor beta (TGF-β), endoglin and TGF-β receptor, chemokine (C-C motif) ligand 2 (CCL2) (also known as monocyte chemoattractant protein 1 (MCP1) and small inducible cytokine A2), integrin αVβ3, integrin αVβ5, integrin α5β1, vascular endothelial cadherin (VE-cadherin) (also known as cadherin 5, type 2, and (Cluster of Differentiation 144 (CD144)), cluster of differentiation 31 (CD31) (also known as platelet endothelial cell adhesion molecule (PECAM-1)), ephrin, plasminogen activator (e.g., Factor XIa, and Factor XIIa), plasminogen activator inhibitor-1, endothelial nitric oxide synthases (eNOS), and cyclooxygenase-2 (COX-2) (also known as prostaglandin-endoperoxide synthase 2), CD133 antigen (also known as prominin-1, and AC133), DNA-binding protein inhibitor ID-1, DNA-binding protein inhibitor ID3, class 3 semaphorin, or a combination thereof.

In some embodiments, a factor administered can be a recombinant factor, a mutant of the factor, a fragment of the factor, or a combination thereof. A "fragment" as used herein can be a portion of a naturally occurring protein. Fragments can have the same or substantially the same amino acid sequence as the naturally occurring protein. "Substantially the same" can mean that an amino acid sequence is largely, but not entirely, the same, but retains at least one functional activity of the sequence to which it is related. In general two amino acid sequences are "substantially the same" or "substantially homologous" if they are at least about 85% identical. Fragments which have different three dimensional structures as the naturally occurring protein are also included. An example of this, is a "pro-form" molecule, such as a low activity proprotein that can be modified by cleavage to produce a mature enzyme with significantly higher activity.

### Diseases, Disease Symptoms, and Clinical Outcomes

In some embodiments, thereby exudation or neovascularization in the choroid or retina of the eye of the subject is reduced. The method can comprise determining the exudation in the choroid or retina of the eye of the subject is reduced using optical coherence tomography (OCT) and/or fluorescein angiography. The method can comprise determining the neovascularization in the choroid or retina of the eye of the subject is reduced using optical coherence tomography angiography (OCT-A), indocyanine green (ICG) angiography, or a combination thereof. The increase of non-leaking or minimally-leaking choroidal perfusion and/or non-leaking and/or minimally-leaking retinal perfusion in the subject can be different. In some embodiments, thereby non-leaking or minimally-leaking choroidal perfusion and/or non-leaking and/or minimally-leaking retinal perfusion increases in the subject by, by about, by at least, by at least about, by at most, or by at most about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between two of these numbers.

In some embodiments, thereby new non-leaking or minimally-leaking blood vessels are formed in the choroid or the retina of the eye of the subject. The method can comprise determining the formation of new blood vessels using optical coherence tomography angiography (OCT-A). The method can comprise determining minimal or no exudation from the new blood vessels is minimal using optical coherence tomography (OCT) and/or fluorescein angiography, which indicates the new blood vessels formed are non-leaking or minimally-leaking. The new non-leaking or minimally-leaking blood vessels formed in the choroid or the retina of the eye of the subject can cover different percentage of the macular region of the eye of the subject. In some embodiments, the new non-leaking or minimally-leaking blood vessels formed in the choroid or the retina of the eye of the subject covers, covers about, covers at least, covers at least about, covers at most, or covers at most about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between two of these numbers, of the macular region of the eye of the subject. The new non-leaking or minimally-leaking blood vessels formed in the choroid or retina of the eye of the subject can cover different percentage of the peripheral choroid or peripheral retina of the eye of the subject. In some embodiments, the new non-leaking or minimally-leaking blood vessels formed in the choroid or retina of the eye of the subject covers, covers about, covers at least, covers at least about, covers at most, or covers at most about, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 100%, or a number or a range between two of these numbers, of the peripheral choroid or peripheral retina of the eye of the subject. The visual acuity of the subject can stabilize or improve. Choroidal hypoxia and/or retinal hypoxia can be mitigated in the subject.

In some embodiments, thereby a hypoxia inducible factor (HIF)-mediated blinding complication is mitigated. The HIF-mediated visual loss complication can comprise choroidal neovascularization (CNV), retinal neovascularization, macular edema, or a combination thereof. In some embodiments, thereby retinal edema, subretinal fluid, or both, are reduced. In some embodiments, thereby leaky choroidal neovascularization is mitigated in the subject. In some embodiments, macular atrophy, geographic atrophy (GA), or both are mitigated in the subj ect.

### Routes of Administration

In some embodiments, the administering comprises administering the formulation intravitreally. The administering can comprise administering the formulation subretinally. The administering can comprise administering the formulation to the suprachoroidal space of an eye of the subject. The administering can comprise administering the formulation to the macular region of an eye of the subject. The administering can comprise administering the formulation to one or more retinal or choroidal regions, of the eye of the subject, with reduced perfusion. The administering can comprise administering the formulation to the subject about once every week to about once every year.

As disclosed herein, the patient can be treated by, for example, receiving administration of the formulation. The administration can be for example, once per day, once per two days, once per three days, once per week once per ten days, once per fifteen days, once per month, once per two months, once per three months, once per six months, once per year, once per two years, or a frequency or a range of frequency between any two of these values. The duration that the patient can be treated can vary. For example, the duration of the treatment can be 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 25 months, 26 months, 27 months, 28 months, 29 months, 30 months, 31 months, 32 months, 33 months, 34 months, 35 months, 36 months, four years, five years, six years, seven years, eight years, nine years, 10 years, or a number or a range between any two of these values.

The administering can comprise different numbers of total administrations. In some embodiments, the administering can comprise, comprise about, comprise at least, comprise at least about, comprise at most, or comprise at most about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values.

The administering can comprise different numbers of courses of treatment. In some embodiments, the administering can comprise, comprise about, comprise at least, comprise at least about, comprise at most, or comprise at most about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, of courses of treatment.

A course of treatment can include different numbers of administrations in different implementations. In some embodiments, a course of treatment can comprise, comprise about, comprise at least, comprise at least about, comprise at most, or comprise at most about, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, or a number or a range between any two of these values, of administrations.

### Monitoring

In some embodiments, the method comprises: using optical coherence tomography angiography (OCT-A) and optical coherence tomography (OCT) to determine choroidal or retinal revascularization or non-leaking or minimally-leaking choroidal or retinal revascularization in the subject, respectively; and if adequate, discontinuing temporarily or permanently administering the formulation to the subject. In some embodiments, the method comprises: using optical coherence tomography angiography (OCT-A), fluorescein angiography and/or optical coherence tomography (OCT) to determine choroidal or retinal revascularization or non-leaking or minimally-leaking choroidal or retinal revascularization in the subject, respectively; and if inadequate, continuing administering the formulation to the subject. In some embodiments, the method comprises: using optical coherence tomography angiography (OCT-A), fluorescein angiography and/or optical coherence tomography (OCT) to determine choroidal or retinal revascularization or non-leaking or minimally-leaking choroidal or retinal revascularization in the subject, respectively; and if excessive, administering an antagonist of a second pro-angiogenic factor to the subject. The antagonist of the second pro-angiogenic factor can comprise an antibody targeting the second pro-angiogenic factor. The determining can comprise performing an ocular examination or sequential ocular examinations. The sequential ocular examinations can comprise visual acuity assessment, a fundus auto-fluorescence (FAF) examination, an optical coherence tomography (OCT) examination, an optical coherence tomography angiography (OCT-A) examination, a fluorescein angiography (FA) examination, indocyanine green (ICG) angiography examination, or a combination thereof. In some embodiments, the method comprises: determining vascular maturation in the subject using optical coherence tomography angiography (OCT), fluorescein angiography (FA), indocyanine green (ICG) angiography, or a combination thereof; and if inadequate, administering the vascular maturation factor to the subject. The determining can comprise determining the extent of vascular maturation using optical coherence tomography (OCT) or fluorescein and indocyanine green (ICG) angiography. The method can comprise: using optical coherence tomography angiography (OCT-A) to determine choroidal revascularization in the subject.

### Effective Amount

The therapeutically effective amount of the pro-angiogenic factor can be different in different implementations. In some embodiments, the therapeutically effective amount of the formulation is, is about, is at least, is at least about, is at most, or is at most about, 0.001 mg, 0.002 mg, 0.003 mg, 0.004 mg, 0.005 mg, 0.006 mg, 0.007 mg, 0.008 mg, 0.009 mg, 0.01 mg, 0.02 mg, 0.03 mg, 0.04 mg, 0.05 mg, 0.06 mg, 0.07 mg, 0.08 mg, 0.09 mg, 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, or a number or a range between any two of these values, of the pro-angiogenic factor per administering.

The therapeutically effective amount of the vascular maturation factor per administering can be different in different implementations. In some embodiments, the therapeutically effective amount of the formulation is, is about, is at least, is at least about, is at most, or is at most about, 0.001 mg, 0.002 mg, 0.003 mg, 0.004 mg, 0.005 mg, 0.006 mg, 0.007 mg, 0.008 mg, 0.009 mg, 0.01 mg, 0.02 mg, 0.03 mg, 0.04 mg, 0.05 mg, 0.06 mg, 0.07 mg, 0.08 mg, 0.09 mg, 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, or a number or a range between any two of these values, of the vascular maturation factor per administering.

The therapeutically effective amount of the factor (e.g., an angiogenesis or angiogenic factor or agent, or a growth factor) per administering can be different in different implementations. In some embodiments, the therapeutically effective amount of the factor is, is about, is at least, is at least about, is at most, or is at most about, 0.001 mg, 0.002 mg, 0.003 mg, 0.004 mg, 0.005 mg, 0.006 mg, 0.007 mg, 0.008 mg, 0.009 mg, 0.01 mg, 0.02 mg, 0.03 mg, 0.04 mg, 0.05 mg, 0.06 mg, 0.07 mg, 0.08 mg, 0.09 mg, 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, or a number or a range between any two of these values, of the factor per administering.

### Composition and Formulation

Disclosed herein include embodiments of a composition or formulation comprising a pro-angiogenic factor and/or a vascular maturation factor for use in increasing non-leaking or minimally-leaking choroidal perfusion or non-leaking or minimally-leaking retinal perfusion in a subject in need thereof. Disclosed herein include embodiments of a composition or formulation comprising a pro-angiogenic factor and/or a vascular maturation factor for use in the treatment of an ocular disease associated with, or characterized by, choroidal hypoperfusion or retinal hypoperfusion in a subject in need thereof. Disclosed herein include embodiments of a composition or formulation comprising an angiogenesis or angiogenic factor or agent for use in increasing non-leaking or minimally-leaking choroidal perfusion or non-leaking or minimally-leaking retinal perfusion in a subject in need thereof. Disclosed herein include embodiments of a composition or formulation comprising an angiogenesis factor for use in the treatment of an ocular disease associated with, or characterized by, choroidal hypoperfusion or retinal hypoperfusion in a subject in need thereof. The angiogenesis or angiogenic factor or agent can be, or can comprise, a pro-angiogenic factor and/or a vascular maturation factor. The composition or formulation can comprise a sustained release composition or formulation of the pro-angiogenic factor and the vascular maturation factor.

The formulation or composition can comprise different concentrations of the pro-angiogenic factor in different implementations. In some embodiments, the concentration of the pro-angiogenic factor in the formulation or composition is, is about, is at least, is at least about, is at most, or is at most about, 0.001 mg/ml, 0.002 mg/ml, 0.003 mg/ml, 0.004 mg/ml, 0.005 mg/ml, 0.006 mg/ml, 0.007 mg/ml, 0.008 mg/ml, 0.009 mg/ml, 0.01 mg/ml, 0.02 mg/ml, 0.03 mg/ml, 0.04 mg/ml, 0.05 mg/ml, 0.06 mg/ml, 0.07 mg/ml, 0.08 mg/ml, 0.09 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, 50 mg/ml, 51 mg/ml, 52 mg/ml, 53 mg/ml, 54 mg/ml, 55 mg/ml, 56 mg/ml, 57 mg/ml, 58 mg/ml, 59 mg/ml, 60 mg/ml, 61 mg/ml, 62 mg/ml, 63 mg/ml, 64 mg/ml, 65 mg/ml, 66 mg/ml, 67 mg/ml, 68 mg/ml, 69 mg/ml, 70 mg/ml, 71 mg/ml, 72 mg/ml, 73 mg/ml, 74 mg/ml, 75 mg/ml, 76 mg/ml, 77 mg/ml, 78 mg/ml, 79 mg/ml, 80 mg/ml, 81 mg/ml, 82 mg/ml, 83 mg/ml, 84 mg/ml, 85 mg/ml, 86 mg/ml, 87 mg/ml, 88 mg/ml, 89 mg/ml, 90 mg/ml, 91 mg/ml, 92 mg/ml, 93 mg/ml, 94 mg/ml, 95 mg/ml, 96 mg/ml, 97 mg/ml, 98 mg/ml, 99 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 260 mg/ml, 270 mg/ml, 280 mg/ml, 290 mg/ml, 300 mg/ml, 310 mg/ml, 320 mg/ml, 330 mg/ml, 340 mg/ml, 350 mg/ml, 360 mg/ml, 370 mg/ml, 380 mg/ml, 390 mg/ml, 400 mg/ml, 410 mg/ml, 420 mg/ml, 430 mg/ml, 440 mg/ml, 450 mg/ml, 460 mg/ml, 470 mg/ml, 480 mg/ml, 490 mg/ml, 500 mg/ml, or a number or a range between any two of these values.

The formulation or composition can comprise different concentrations of the vascular maturation factor in different implementations. In some embodiments, the concentration of the vascular maturation factor in the formulation or composition is, is about, is at least, is at least about, is at most, or is at most about, 0.001 mg/ml, 0.002 mg/ml, 0.003 mg/ml, 0.004 mg/ml, 0.005 mg/ml, 0.006 mg/ml, 0.007 mg/ml, 0.008 mg/ml, 0.009 mg/ml, 0.01 mg/ml, 0.02 mg/ml, 0.03 mg/ml, 0.04 mg/ml, 0.05 mg/ml, 0.06 mg/ml, 0.07 mg/ml, 0.08 mg/ml, 0.09 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, 50 mg/ml, 51 mg/ml, 52 mg/ml, 53 mg/ml, 54 mg/ml, 55 mg/ml, 56 mg/ml, 57 mg/ml, 58 mg/ml, 59 mg/ml, 60 mg/ml, 61 mg/ml, 62 mg/ml, 63 mg/ml, 64 mg/ml, 65 mg/ml, 66 mg/ml, 67 mg/ml, 68 mg/ml, 69 mg/ml, 70 mg/ml, 71 mg/ml, 72 mg/ml, 73 mg/ml, 74 mg/ml, 75 mg/ml, 76 mg/ml, 77 mg/ml, 78 mg/ml, 79 mg/ml, 80 mg/ml, 81 mg/ml, 82 mg/ml, 83 mg/ml, 84 mg/ml, 85 mg/ml, 86 mg/ml, 87 mg/ml, 88 mg/ml, 89 mg/ml, 90 mg/ml, 91 mg/ml, 92 mg/ml, 93 mg/ml, 94 mg/ml, 95 mg/ml, 96 mg/ml, 97 mg/ml, 98 mg/ml, 99 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 260 mg/ml, 270 mg/ml, 280 mg/ml, 290 mg/ml, 300 mg/ml, 310 mg/ml, 320 mg/ml, 330 mg/ml, 340 mg/ml, 350 mg/ml, 360 mg/ml, 370 mg/ml, 380 mg/ml, 390 mg/ml, 400 mg/ml, 410 mg/ml, 420 mg/ml, 430 mg/ml, 440 mg/ml, 450 mg/ml, 460 mg/ml, 470 mg/ml, 480 mg/ml, 490 mg/ml, 500 mg/ml, or a number or a range between any two of these values.

The formulation or composition can comprise different concentrations of the factor (e.g., an angiogenesis or angiogenic factor or agent, or a growth factor) in different implementations. In some embodiments, the concentration of the factor in the formulation or composition is, is about, is at least, is at least about, is at most, or is at most about, 0.001 mg/ml, 0.002 mg/ml, 0.003 mg/ml, 0.004 mg/ml, 0.005 mg/ml, 0.006 mg/ml, 0.007 mg/ml, 0.008 mg/ml, 0.009 mg/ml, 0.01 mg/ml, 0.02 mg/ml, 0.03 mg/ml, 0.04 mg/ml, 0.05 mg/ml, 0.06 mg/ml, 0.07 mg/ml, 0.08 mg/ml, 0.09 mg/ml, 0.1 mg/ml, 0.2 mg/ml, 0.3 mg/ml, 0.4 mg/ml, 0.5 mg/ml, 0.6 mg/ml, 0.7 mg/ml, 0.8 mg/ml, 0.9 mg/ml, 1 mg/ml, 2 mg/ml, 3 mg/ml, 4 mg/ml, 5 mg/ml, 6 mg/ml, 7 mg/ml, 8 mg/ml, 9 mg/ml, 10 mg/ml, 11 mg/ml, 12 mg/ml, 13 mg/ml, 14 mg/ml, 15 mg/ml, 16 mg/ml, 17 mg/ml, 18 mg/ml, 19 mg/ml, 20 mg/ml, 21 mg/ml, 22 mg/ml, 23 mg/ml, 24 mg/ml, 25 mg/ml, 26 mg/ml, 27 mg/ml, 28 mg/ml, 29 mg/ml, 30 mg/ml, 31 mg/ml, 32 mg/ml, 33 mg/ml, 34 mg/ml, 35 mg/ml, 36 mg/ml, 37 mg/ml, 38 mg/ml, 39 mg/ml, 40 mg/ml, 41 mg/ml, 42 mg/ml, 43 mg/ml, 44 mg/ml, 45 mg/ml, 46 mg/ml, 47 mg/ml, 48 mg/ml, 49 mg/ml, 50 mg/ml, 51 mg/ml, 52 mg/ml, 53 mg/ml, 54 mg/ml, 55 mg/ml, 56 mg/ml, 57 mg/ml, 58 mg/ml, 59 mg/ml, 60 mg/ml, 61 mg/ml, 62 mg/ml, 63 mg/ml, 64 mg/ml, 65 mg/ml, 66 mg/ml, 67 mg/ml, 68 mg/ml, 69 mg/ml, 70 mg/ml, 71 mg/ml, 72 mg/ml, 73 mg/ml, 74 mg/ml, 75 mg/ml, 76 mg/ml, 77 mg/ml, 78 mg/ml, 79 mg/ml, 80 mg/ml, 81 mg/ml, 82 mg/ml, 83 mg/ml, 84 mg/ml, 85 mg/ml, 86 mg/ml, 87 mg/ml, 88 mg/ml, 89 mg/ml, 90 mg/ml, 91 mg/ml, 92 mg/ml, 93 mg/ml, 94 mg/ml, 95 mg/ml, 96 mg/ml, 97 mg/ml, 98 mg/ml, 99 mg/ml, 100 mg/ml, 110 mg/ml, 120 mg/ml, 130 mg/ml, 140 mg/ml, 150 mg/ml, 160 mg/ml, 170 mg/ml, 180 mg/ml, 190 mg/ml, 200 mg/ml, 210 mg/ml, 220 mg/ml, 230 mg/ml, 240 mg/ml, 250 mg/ml, 260 mg/ml, 270 mg/ml, 280 mg/ml, 290 mg/ml, 300 mg/ml, 310 mg/ml, 320 mg/ml, 330 mg/ml, 340 mg/ml, 350 mg/ml, 360 mg/ml, 370 mg/ml, 380 mg/ml, 390 mg/ml, 400 mg/ml, 410 mg/ml, 420 mg/ml, 430 mg/ml, 440 mg/ml, 450 mg/ml, 460 mg/ml, 470 mg/ml, 480 mg/ml, 490 mg/ml, 500 mg/ml, or a number or a range between any two of these values.

In some embodiments, revascularization resulted from administering the composition or formulation disclosed herein can be used for mitigation of GA and CNV in AMD. In some embodiments, revascularization resulted from administering the composition or formulation disclosed herein can be used to treat other causes of choroidal neovascularization and macular atrophy, such as seen in degenerative myopia.

### Kits

Disclosed herein include embodiments of a kit comprising: a formulation comprising a pro-angiogenic factor and/or a vascular maturation factor; and a label indicating that the formulation is for increasing choroidal perfusion or retinal perfusion. Disclosed herein include embodiments of a kit comprising: a formulation comprising a pro-angiogenic factor and/or a vascular maturation factor; and a label indicating that the formulation is for treating an ocular disease associated with, or characterized by, choroidal hypoperfusion and/or retinal hypoperfusion. Disclosed herein include embodiments of a kit comprising: a formulation comprising an angiogenesis factor; and a label indicating that the formulation is for increasing choroidal perfusion and/or retinal perfusion. Disclosed herein include embodiments of a kit comprising: a formulation comprising an angiogenesis factor; and a label indicating that the formulation is for treating an ocular disease associated with, or characterized by, choroidal hypoperfusion and/or retinal hypoperfusion. The angiogenesis factor can be, or can comprise, a pro-angiogenic factor and/or a vascular maturation factor.

In some embodiments, the label indicates the formulation is for treating a disease selected from a group comprising dry age-related macular degeneration (AMD) and/or geographic atrophy (GA), or a combination thereof. In some embodiments, the label indicates the formulation is for treating a disease selected from a group comprising wet age-related macular degeneration (AMD), choroidal neovascularization (CNV), polypoidal choroidal vasculopathy, degenerative (pathologic) myopia, giant cell arteritis, or a combination thereof. In some embodiments, the label indicates the formation is for treating a disease selected from a group comprising diabetic macular edema, macular edema from retinal vein occlusion, diabetic retinopathy, retinopathy of prematurity, retinal vein occlusion, retinal neovascularization in diabetes, optic nerve neovascularization in diabetes, familial exudative vitreoretinopathy, sickle cell disease, or a combination thereof. Non-leaking or minimally-leaking retinal perfusion in the subject can increase after the formulation is administered to the subject. Non-leaking or minimally-leaking retinal revascularization in the subject can be promoted after the formulation is administered to the subject. Hypoxia in the retina of the eye of the subject can be reduced, and/or ischemia in the retina of the eye of the subject can be reduced after the formulation is administered to the subject. Non-leaking or minimally-leaking retinal perfusion in the subject can increase after the formulation is administered to the subject.

In some embodiments, the label indicates the formulation is for treating a disease selected from a group comprising radiation retinopathy, radiation optic neuropathy, or a combination thereof. The label can indicate the formulation is for treating the subject after the subject receives a radiation treatment for a disease selected from a group comprising an intraocular tumor a head tumor, a neck tumor, or a combination thereof, resulting in delayed onset of the disease. The label can indicate the administration of the formulation to the subject occurs about 1-26 weeks after the subject receives the radiation treatment. In some embodiments, the label indicates the formulation is for treating a hypoxia inducible factor (HIF)-mediated blinding complication. The HIF-mediated visual loss complication comprises choroidal neovascularization (CNV), retinal neovascularization, macular edema, or a combination thereof.

### EXAMPLES

Some aspects of the embodiments discussed above are disclosed in further detail in the following examples, which are not in any way intended to limit the scope of the present disclosure.

### Example 1

### Non-leaking choroidal neovascularization protects overlying tissue from developing geographic atrophy:

There is clinical support for the hypothesis that a mature neovascular network underneath the fovea can support normal foveal architecture and prevent development of geographic atrophy. OCT angiography has shown that patients with type 1 CNV (sub-RPE) underneath the fovea develop GA eccentric to the retina overlying the neovascularization, suggesting that the CNV maintains the viability of the overlying macular tissue.

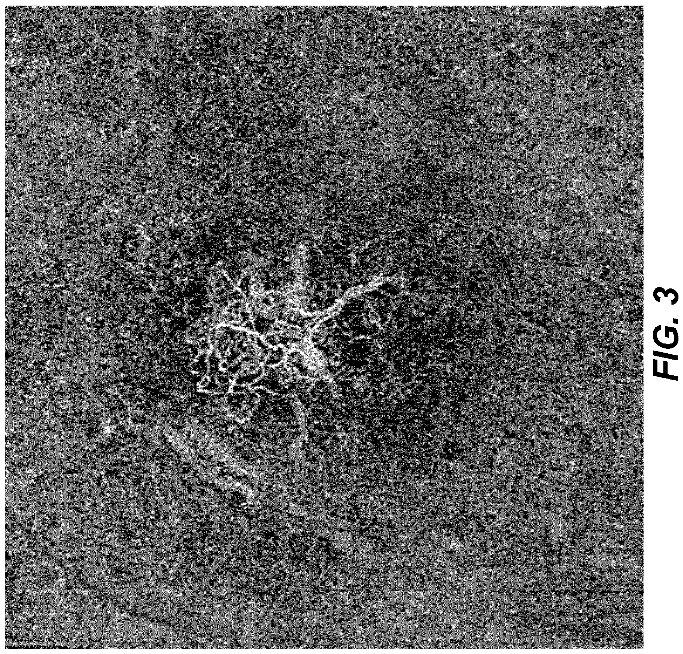

An example of this is shown below in an 88 year-old subject with disciform scar OS. The subject had received numerous injections in OD until he got post-injection endophthalmitis one year previous to the OCT angiography study shown in FIG. 3. The OCT and OCT angiogram reveal subfoveal CNV that is non-leaking (Let's show a picture of the OCT, too; don't' have it.). Then, he did not want to have further injections. He has had no exudation nor progression of atrophy into the fovea noted for over one year. Vision 20/25- OD.

### Example 2

### AMD with progressively expanding extrafoveal geographic atrophy (GA)

A patient with AMD, good central visual acuity (≥20/40) and early GA that spares fixation is considered for treatment with pro-angiogenic therapy. OCTA examination reveals macular flow voids indicating reduced choriocapillaris perfusion. Pro-angiogenic compound(s) is/are administered intravitreally. Alternatively, pro-angiogenic therapy can be administered by a suprachoroidal route or injected into the subretinal space in the macular region. Following pro-angiogenic therapy, the patient is followed by sequential ocular examination, including visual acuity assessment, fundus auto-fluorescence (FAF), OCT and OCTA examinations. On follow up, GA progression is halted or slowed as demonstrated by serial FAF and OCT examination. OCTA demonstrates reduction in macular flow voids, including at the margin of the existing GA lesion(s). If during follow-up there is progressive choriocapillaris attrition with progression of existing GA lesions or development of new lesions, then pro-angiogenic therapy is re-administered as above.

### Example 3

### AMD with CNV

OCTA in patients with CNV shows increased flow voids (choriocapillaris perfusion defects) surrounding the neovascular membrane. Resultant hypoxia results in VEGF secretion by RPE with subsequent CNV development. After treatment with anti-VEGF agents, CNV usually shows reduction in permeability. To minimize exudation and visual loss, repeated anti-VEGF injections are required. As an alternative, angiogenic factors that promote non-leaking choroidal neovascularization, reduce ischemia and resultant need for frequent VEGF injections. Pro-angiogenic choroidal revascularization therapy reconstitutes choriocapillaris and eliminates the ischemic drive for VEGF secretion. A patient with wet AMD is initially treated with an anti-VEGF agent that stops leakage from choroidal neovascularization. At the same time or shortly thereafter, a pro-angiogenic factor is administered intravitreally or subretinally, or in the suprachoroidal space, which promotes non-leaking choriocapillaris angiogenesis. Growth of these new vessels eliminates or minimizes the ischemic drive for VEGF secretion. The CNV is thus prevented from leaking further because VEGF secretion in diminished in the absence of significant ischemia.

### Example 4

### Treatment of diabetic macular edema

Patients with diabetic macular edema are treated with anti-VEGF agents to reduced vascular permeability of retinal capillaries induced by VEGF. VEGF secretion in diabetic macular edema is mediated by capillary non-perfusion and resultant hypoxia. Control of diabetic macular edema initially requires monthly injections of an anti-VEGF agent to reduce exudation and maximize visual acuity. As an alternative to repeated injections an anti-VEGF agent, pro-angiogenic factor(s) is/are injected to revascularize the retinal capillaries of the ischemic macula. By revascularizing of the retinal capillary bed with non-leaking capillaries, hypoxia is reversed, VEGF secretion diminished, and the macular edema resolves. As in the case of wet AMD, revascularization therapy can be complemented by use of anti-VEGF agents to minimize vascular leakage during the time when new retinal capillaries are growing. The same principles of retinal revascularization with non-leaking capillaries would hold in cases of macular edema from retinal vein occlusions.

### Example 5

### Posterior segment and anterior segment neovascularization

When severe retinal ischemia occurs in diabetic retinopathy or after retinal vein occlusion, retinal or anterior segment neovascularization can result. Untreated, these forms of neovascularization can cause severe visual loss from vitreous hemorrhage, retinal detachment, or neovascular glaucoma. Treatment of retinal or anterior segment neovascularization relies on anti-VEGF agents and/or laser photocoagulation. As an alternative, patients with proliferative diabetic retinopathy are treated with pro-angiogenic factor(s) that revascularize the ischemic retina. This reduces hypoxia, VEGF secretion, and resultant neovascularization. In certain cases, pro-angiogenic therapy can be used in concert with anti-VEGF agents or laser photocoagulation to stabilize the retina while non- or minimally leaking angiogenesis occurs with pro-angiogenic factor(s). The pro-angiogenic factor(s) are injected intravitreally. The pro-angiogenic factor(s) can be injected preferentially over regions of more severe ischemia as determined by fluorescein angiography or OCTA. Intravitreal pro-angiogenic agents also can be used prophylactically when there is significant loss of retinal capillaries in the absence of frank neovascularization. In this case, use of pro-angiogenic factor(s) can revascularize the ischemic retina and prevent development of neovascularization.

### Example 6

### Radiation retinopathy and radiation optic neuropathy

Radiation treatment of intraocular tumors, most commonly choroidal melanoma, can cause delayed onset of radiation retinopathy and optic neuropathy. Radiation damage to capillary endothelium causes closure and resultant ischemia of the retinal vasculature. Similarly, damage to the pre-laminar and deeper optic nerve capillaries can cause irreversible ischemic damage to the optic nerve. In patients undergoing radiation treatment of intraocular or head and neck tumors in which the eye receives radiation, pro-angiogenic factor(s) are used to revascularize the retina and optic nerve after radiation therapy is complete. Generally, revascularization therapy is administered 1-26 weeks after radiation therapy, since the onset of radiation retinopathy and optic neuropathy are delayed after radiation therapy. By revascularizing and replacing damaged capillaries, this therapy prevents ischemic retina and optic nerve damage and visual loss is mitigated.

### Example 7

### Animal study protocol for oxygen-induced retinopathy of prematurity model

**Protocol title:** Evaluation of an angiogenic agent in the Oxygen-induced Retinopathy of Prematurity Model.

### Introduction of murine model of retinopathy of prematurity (muROP):

The development of a reproducible and quantifiable model of retinal neovascularization and the proliferative phase of retinopathy of prematurity in mice has been described. This model approximates conditions of human premature neonates who can develop retinopathy of prematurity (ROP).

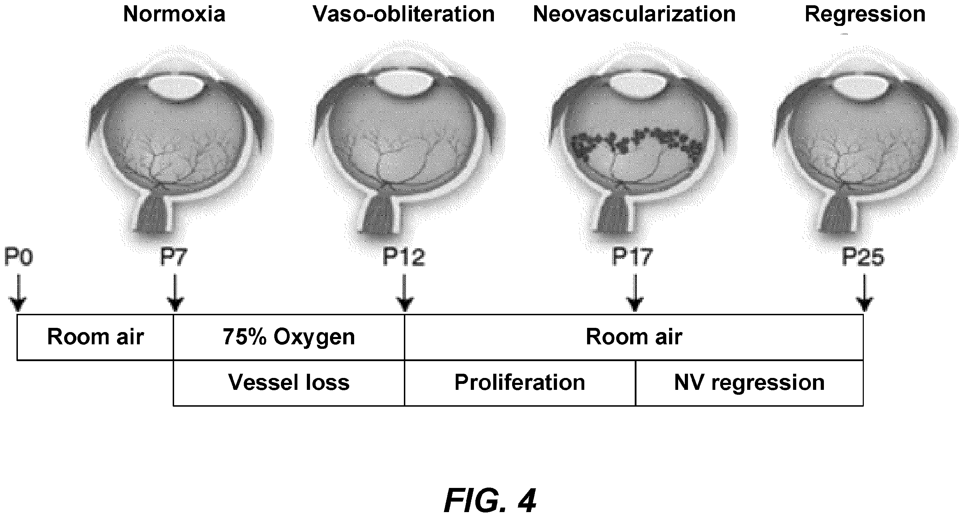

In this model, illustrated in FIG. 4A, neonatal mice (C57BL/6j) and their mothers are exposed to 5 days of hyperoxia (75% oxygen controlled by an incubator and an oxygen blender) starting at postnatal day 7 (P7). At postnatal day 12, the neonates and mothers are returned to room air. P7 mice exposed to 75% oxygen for 5 days resulted in reproducible and quantifiable retinal neovascularization without hypertrophy or dilatation of the hyaloid vessels. The greatest neovascular response occurred from P17-P21, followed by slow regression of the new vessels with reestablishment of a more normal branching vascular pattern, visible in retinal flat-mounts by P24. It has been shown that hyperoxia results in a reduction of VEGF, development of retinal capillary non-perfusion; and, that reintroduction of room air is associated with the exuberant overproduction of VEGF which drives retinal neovascularization.

In neonates with ROP, treating with anti-VEGF reduces neovascularization, but retinopathy can recur. Treating with an angiogenic agent alone or in combination with VEGF at the beginning, during, or after of the hyperoxia period can result in revascularization with non-leaky vessels and mitigation of retinopathy.

**Objectives:** 1) To evaluate the effect of treatment with an angiogenic agent beginning at postnatal day 7, 10 or 12 (P7, 10 or 12) on retinal vessels (assessed by optical coherence tomography angiography, OCTA) in the murine model of Oxygen-induced Retinopathy of Prematurity.

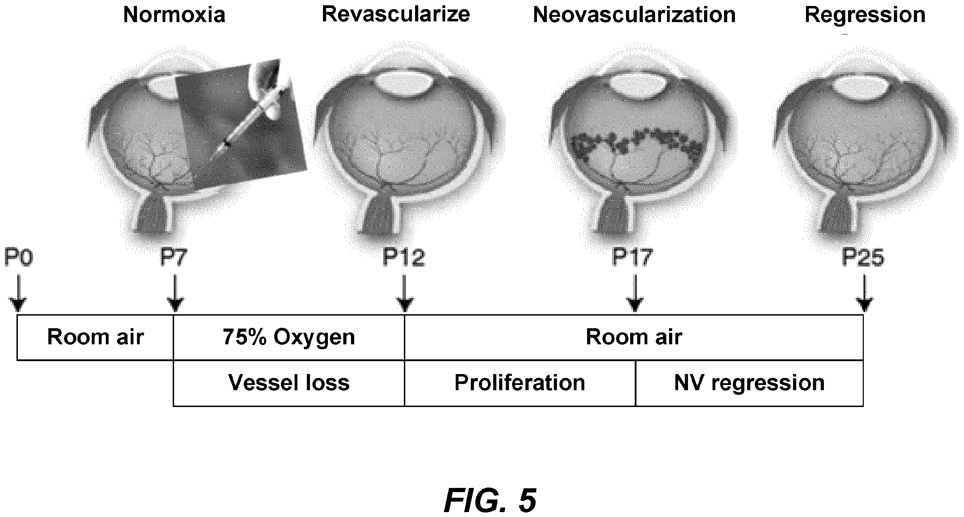

**Methodology:** This study consists of 2-3 pilot studies, followed by a POC study in C57B/6 neonatal mice. P7 mice and their nursing mothers will be randomized into 3-4 treatment groups (depending on pilot sub-studies) and treated with 75% O₂ for 5 days. An angiogenic agent alone, angiogenic agent plus VEGF, VEGF (control) or NS (control) will be administered by intra-vitreal (IVT) injection into one eye of each mouse on P7, P 10, P12, or P14 (FIG. 5). The companion eye will not be treated. On day P12, mothers and pups will be returned to room air. Animals will be evaluated by OCTA at P7, P12 (maximum vessel drop-out), and P17 (maximum neovascularization).
**Number of animals for this study:** 66 Neonatal mice

### Diagnosis and Main Criteria for Inclusion (for human clinical phase):

**Number of treatments:** 1
**Dosing for each treatment:** 3 dose levels of angiogenic agent (TBD in pilot substudies)
**Duration of each treatment:** Single treatment
**Criteria for Evaluation:** percent vascular non-perfusion as determined by OCTA.

Statistical Methods Based on the difference observed in eyes without Diabetic Retinopathy (DR), those with moderate non-proliferative DR and those with proliferative DR, a decrease in vascular non-perfusion of at least 40% is a reasonable expectation (Silva, 2015). Pilot studies will be used to determine the difference between angiogenic treatment vs no treatment in the mice with oxygen induced retinopathy. The subsequent efficacy studies will be sized based on this information.

**Measures:** OCTA, Fluorescein Angiography (FA), retinal whole mounts for pericytes.

**Safety:** No adverse events, such as intraocular inflammation, due to study drug in POC efficacy studies and no aberrant vessels.

### Efficacy:

a. Demonstrate a statistically significant improvement in retinal blood flow compared to vehicle-treated controls:
   i. Demonstrate improvement in retinal blood flow using OCTA, using published protocols,
   ii. Corroborate OCT-A results using retinal vessel wholemounts.
b. Demonstrate that new vessels generated by angiogenic agent are not leaky:
   i. Reduction of fluorescein leakage by at least 50% compared to vehicle-treated controls, using fluorescence angiography,
   ii. Corroborate lack of leakiness using co-staining of retinal wholemounts for pericytes.

**Pharmaco-kinetics:** None

### Example 8

### Animal study protocol for sodium iodate-induced retinopathy

### Protocol title: Evaluation of an angiogenic agent in the Sodium-Iodate (SI,NaIO₃)-induced Retinopathy model

### Introduction of the murine model of SI-induced Retinopathy:

The course of SI-induced retinal degeneration in mice has been described. This model approximates the retinal degeneration that occurs in human adult macular degeneration (AMD) and other outer retinal degenerations.

In this model a single dose of SI 10-50 mg/ml is given by intraperitoneal (IP) injection to 7 to 8-week old BALB/c (albino) and C57Bl/6j (pigmented) mice and results in reproduceable dose-dependent retinal pigment epithelium (RPE) injury followed by vision impairment, dysfunction, and loss of photoreceptors in both mice strains. SI is a chemical oxidizing agent that primarily affects the RPE, causing subsequent damage to photoreceptors and choriocapillaris.

A 50 mg/ml dose of SI given IP induces reproduceable RPE injury without systemic toxicity. Time-dependent deterioration in retinal function and morphology consistently occurs between 1 to 4 weeks after SI injections as measured by electroretinography (ERG) responses, thinning of retinal layers on optical coherence tomography (OCT), histology, and loss of RPE nuclei. Alternative models in a variety of species have been described where SI is administered by intraperitoneal, intravenous, subretinal, or by retrobulbar injection with similar results.

**Objectives:** 1) To evaluate the effect of treatment with an angiogenic agent on choriocapillaris perfusion (assessed by optical coherence tomography angiography, OCTA) in the murine model of SI-induced retinal damage.

**Methodology:** This study consists of 2-3 pilot studies, followed by a POC efficacy study in C57B/6j or BALB/c mice. C57BL/6j or BALB/c mice (7-8-weeks of age) will be randomized into 3 or 4 groups and injected with SI 10-50 mg/ml IP and/or 1 microliter of 5 mg/cc subretinally (route to be determined by pilot studies). At 1 week, 2 weeks and 4 weeks, mice will then be treated with an angiogenic agent alone or in combination with VEGF, with VEGF (control) or with saline (control). The companion eye will not be treated. Eyes will be evaluated by OCTA at baseline and weeks 1, 2, 3, 4, and 6, Fluorescein Angiography (FA) at baseline, Week 4, and Week 6, and retinal wholemounts of all eyes will be obtained at the end of the study.
**Number of animals for this study:** approximately 100 mice
**[0158] Number of treatments:** 1 to 3 (TBD in pilot studies)
**Dosing for each treatment:** 3 dose levels of angiogenic agent (TBD in pilot studies)
**Duration of each treatment:** Single treatment or multiple treatments will be determined in pilot studies.

### Criteria for Evaluation:

a. Percent choriocapillaris flow voids determined by OCTA using pre-approved OCTA protocols.
b. Percent of fluorescein leakage compared to vehicle-treated controls, using fluorescence angiography

### Statistical Methods:

a. At least 40% reduction of choriocapillaris flow voids, using pre-approved OCTA protocols, within 2 degrees of geographic atrophy, generated by SI administration. Pilot studies will be used to determine variability of OCTA for evaluating choriocapillaris flow voids in the SI model and this information will be used for sample size determination for the efficacy study.
b. At least a 50% reduction in fluorescein leakage compared to saline controls using fluorescein angiography
c. Lack of leakiness using co-staining of retinal wholemounts for pericytes will be described

**Measures:** OCTA, Fluorescein Angiography (FA), retinal whole mounts for pericytes.

**Safety:** No adverse events (i.e., inflammation, closure of blood vessels, and aberrant vessels) due to study drug in POC efficacy studies.

### Efficacy:

a. Demonstrate a statistically significant improvement in choroidal blood flow compared to vehicle-treated controls:
   i. Reduction of choriocapillaris (CC) flow voids within 2 degrees of geographic atrophy generated by sodium iodate administration by at least 40% using OCTA pre-approved protocols.
   ii. Corroborate increase in choriocapillaris using quantification of endothelial cell-stained choroidal vessel wholemounts
b. Demonstrate that new vessels generated by angiogenic agent are not leaky:
   i. Reduction of fluorescein leakage by at least 50% compared to vehicle-treated controls, using fluorescein angiography
   ii. Corroborate lack of leakiness using co-staining of retinal wholemounts for pericytes

**Pharmaco-kinetics:** None

### Terminology

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g*., bodies of the appended claims) are generally intended as "open" terms (*e.g.*, the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.*, "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.*, the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (e.g., " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.*, " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

### CLAUSES

The present application is a divisional application based on an earlier European Patent Application No. 19852434.0, which was in turn derived from PCT Application No. PCT/US2019/047360. The following numbered clauses, which correspond to the claims of that earlier PCT Application as filed, form part of the present disclosure and in particular form further aspects of the invention, whether or not they appear in the present claims.
1. A method for increasing choroidal perfusion in a subject in need thereof, comprising:
   administering a formulation comprising a therapeutically effective amount of a pro-angiogenic factor and/or a vascular maturation factor to the subject in need of increased choroidal perfusion.
2. The method of clause 1, thereby non-leaking or minimally-leaking choroidal perfusion in the subject is increased.
3. A method for promoting non-leaking or minimally-leaking choroidal revascularization in a subject in need thereof, comprising:
   administering a formulation comprising a therapeutically effective amount of a pro-angiogenic factor and/or a vascular maturation factor to the subject in need of non-leaking or minimally-leaking choroidal revascularization.
4. The method of clause 3, thereby non-leaking or minimally-leaking choroidal revascularization in the subject is promoted.
5. A method for increasing choroidal perfusion in a subject in need thereof, comprising:
   administering a formulation comprising a therapeutically effective amount of an angiogenesis factor to the subject in need of increased choroidal perfusion.
6. The method of clause 5, thereby non-leaking or minimally-leaking choroidal perfusion in the subject is increased.
7. A method for promoting non-leaking or minimally-leaking choroidal revascularization in a subject in need thereof, comprising:
   administering a formulation comprising a therapeutically effective amount of an angiogenesis factor to the subject in need of non-leaking or minimally-leaking choroidal revascularization.
8. The method of clause 7, thereby non-leaking or minimally-leaking choroidal revascularization in the subject is promoted.
9. The method of any one of clauses 5-8, wherein the angiogenesis factor is a pro-angiogenic factor and/or a vascular maturation factor.
10. A method for increasing choroidal perfusion in a subject in need thereof, comprising:
   causing a level of a pro-angiogenic factor in the choroid of an eye of the subject to increase; and
   administering a therapeutically effective amount of a vascular maturation factor to the subject.
11. The method of clause 10, thereby non-leaking or minimally-leaking choroidal perfusion in the subject is increased.
12. A method for promoting non-leaking or minimally-leaking choroidal revascularization in a subject in need thereof, comprising:
   causing a level of a pro-angiogenic factor in the choroid of an eye of the subject to increase; and
   administering a therapeutically effective amount of a vascular maturation factor to the subject.
13. The method of clause 12, thereby non-leaking or minimally-leaking choroidal revascularization in the subject is promoted.
14. The method of any one of clauses 10-13, wherein causing the level of the pro-angiogenic factor in the choroid of the eye of the subject to increase comprises discontinuing or reducing the administration frequency of an antagonist of a second pro-angiogenic factor.
15. The method of clause 14, wherein the pro-angiogenic factor and the second pro-angiogenic factor are identical.
16. The method of any one of clauses 14-15, wherein the antagonist of the second pro-angiogenic factor comprises an antibody targeting the second pro-angiogenic factor, or a fragment thereof
17. The method of any one of clauses 10-13, wherein causing the level of the second pro-angiogenic factor in the choroid of the eye of the subject to increase comprises administering a therapeutically effective amount of the second pro-angiogenic factor to the subject.
18. The method of any one of clauses 1-17, thereby macular flow voids are reduced, hypoxia in the outer retina and retinal pigment epithelium (RPE) of the eye of the subject is reduced, and/or ischemia in the outer retina and RPE of the eye of the subject is reduced.
19. The method of any one of clauses 1-18, thereby non-leaking or minimally-leaking choroidal perfusion in the subject is increased.
20. The method of any one of clauses 1-19, comprising:
   determining an extent of choroidal perfusion or non-leaking or minimally-leaking choroidal revascularization in the subject to be inadequate; and
   continue administering the formulation comprising the effective amount of the pro-angiogenic factor and/or the vascular maturation factor to the subject.
21. The method of clause 20, wherein the determining comprises performing an ocular examination or sequential ocular examinations.
22. The method of clause 21, wherein the sequential ocular examinations comprise visual acuity assessment, a fundus auto-fluorescence (FAF) examination, an optical coherence tomography (OCT) examination, an optical coherence tomography angiography (OCT-A) examination, a fluorescein angiography (FA) examination, an indocyanine green (ICG) angiography examination, or a combination thereof.
23. The method of any one of clauses 1-22, comprising, prior to the administering, determining the subject is in need of increased choroidal perfusion or non-leaking or minimally-leaking choroidal revascularization with an ocular examination.
24. The method of any one of clauses 1-23 wherein the subject has a disease selected from the group consisting of dry age-related macular degeneration (AMD) and/or geographic atrophy (GA), or a combination thereof.
25. The method of clause 24, thereby the progression of the disease is reversed, halted, or slowed.
26. The method of clause 25, wherein the reversing, halting, or slowing of the progression of the disease is mediated by increased choroidal perfusion and/or non-leaking or minimally-leaking choroidal revascularization.
27. The method of any one of clauses 1-23, wherein the subject has a disease selected from the group consisting of wet age-related macular degeneration (AMD), choroidal neovascularization (CNV), polypoidal choroidal vasculopathy, degenerative (pathologic) myopia, or a combination thereof.
28. The method of clause 27, thereby the progression of the disease is reversed, halted, or slowed.
29. The method of clause 28, wherein the reversing, halting, or slowing of the progression of the disease is mediated by increased choroidal perfusion and/or non-leaking or minimally-leaking choroidal revascularization.
30. The method of any one of clauses 27-29, further comprising administering a therapeutically effective amount of an antagonist of a second pro-angiogenic factor that reduces vascular leakage while non-leaking or minimally-leaking choroidal neovascularization develops.
31. A method for increasing retinal perfusion in a subject in need thereof, comprising:
   administering a formulation comprising a therapeutically effective amount of a pro-angiogenic factor, and/or a vascular maturation factor, to the subject in need of increased retinal perfusion.
32. The method of clause 31, thereby non-leaking or minimally-leaking retinal perfusion in the subject is increased.
33. A method for promoting non-leaking or minimally-leaking retinal revascularization in a subject in need thereof, comprising:
   administering a formulation comprising a therapeutically effective amount of a pro-angiogenic factor and/or a vascular maturation factor, to the subject in need of non-leaking or minimally-leaking retinal revascularization.
34. The method of clause 33, thereby non-leaking or minimally-leaking retinal revascularization in the subject is promoted.
35. A method for increasing retinal perfusion in a subject in need thereof, comprising:
   administering a formulation comprising a therapeutically effective amount of an angiogenesis factor to the subject in need of increased retinal perfusion.
36. The method of clause 35, thereby non-leaking or minimally-leaking retinal perfusion in the subject is increased.
37. A method for promoting non-leaking or minimally-leaking retinal revascularization in a subject in need thereof, comprising:
   administering a formulation comprising a therapeutically effective amount of an angiogenesis factor to the subject in need of non-leaking or minimally-leaking retinal revascularization.
38. The method of clause 37, thereby non-leaking or minimally-leaking retinal revascularization in the subject is promoted.
39. The method of any one of clauses 35-38, wherein the angiogenesis factor comprises a pro-angiogenic factor and/or a vascular maturation factor.
40. A method for increasing retinal perfusion in a subject in need thereof, comprising:
   causing a level of a pro-angiogenic factor in the retina of an eye of the subject in need of increased retinal perfusion to increase; and
   administering a therapeutically effective amount of a vascular maturation factor to the subject.
41. The method of clause 40, thereby non-leaking or minimally-leaking retinal perfusion in the subject is increased.
42. A method for promoting non-leaking or minimally-leaking retinal revascularization in a subject in need thereof, comprising:
   causing a level of a pro-angiogenic factor in the retina of an eye of the subject in need of non-leaking or minimally-leaking retinal revascularization to increase; and
   administering a therapeutically effective amount of a vascular maturation factor to the subject.
43. The method of clause 42, thereby non-leaking or minimally-leaking retinal revascularization in the subject is promoted.
44. The method of any one of clauses 40-43, wherein causing the level of the pro-angiogenic factor in the retina of the eye of the subject to increase comprises discontinuing or reducing the administration frequency of an antagonist of a second pro-angiogenic factor.
45. The method of clause 44, wherein the pro-angiogenic factor and the second pro-angiogenic factor are identical.
46. The method of any one of clauses 44-45, wherein the antagonist of the second pro-angiogenic factor comprises an antibody targeting the second pro-angiogenic factor, or a fragment thereof.
47. The method of any one of clauses 40-42, wherein causing the level of the pro-angiogenic factor in the retina of the eye of the subject to increase comprises administering a therapeutically effective amount of the pro-angiogenic factor to the subject.
48. The method of any one of clauses 31-47, thereby hypoxia in the retina of the eye of the subject is reduced, and/or ischemia in the retina of the eye of the subject is reduced.
49. The method of any one of clauses 31-48, thereby increasing non-leaking or minimally-leaking retinal perfusion in the subject.
50. The method of any one of clauses 31-49, comprising:
   determining an extent of retinal perfusion or non-leaking or minimally-leaking retinal revascularization in the subject to be inadequate; and
   continuing to administer the formulation to the subject.
51. The method of clause 50, wherein the determining comprises performing an ocular examination or sequential ocular examinations.
52. The method of clause 51, wherein the ocular examination or the sequential ocular examinations comprise visual acuity assessment, a fundus auto-fluorescence (FAF) examination, an optical coherence tomography (OCT) examination, an optical coherence tomography angiography (OCT-A) examination, a fluorescein angiography (FA) examination, an indocyanine green (ICG) angiography examination, or a combination thereof.
53. The method of any one of clauses 31-52, comprising, prior to the administering, determining whether the subject is in need of increased non-leaking or minimally-leaking retinal perfusion or non-leaking or minimally-leaking retinal revascularization with an ocular examination.
54. The method of any one of clauses 31-53, wherein the subject has a disease selected from the group consisting of diabetic macular edema, macular edema from retinal vein occlusion, diabetic retinopathy, retinal vein occlusion, retinopathy of prematurity, retinal neovascularization in diabetes, optic nerve neovascularization in diabetes, familial exudative vitreoretinopathy, sickle cell disease, or a combination thereof.
55. The method of clause 54, thereby the progression of the disease is halted or slowed.
56. The method of clause 55, wherein the halting or slowing of the progression of the disease is mediated by increased non-leaking or minimally-leaking retinal perfusion and/or non-leaking or minimally-leaking retinal revascularization.
57. The method of any one of clauses 54-56, further comprising administering a therapeutically effective amount of an antagonist of a second pro-angiogenic factor.
58. The method of any one of clauses 31-57, wherein the subject has a disease selected from the group consisting of radiation retinopathy, radiation optic neuropathy, or a combination thereof.
59. The method of clause 58, thereby the progression of the disease is reversed, halted, or slowed.
60. The method of clause 59, wherein the reversing, halting or slowing of the progression of the disease is mediated by increased non-leaking or minimally-leaking retinal perfusion, non-leaking or minimally-leaking retinal revascularization and/or non-leaking or minimally-leaking revascularization of the optic nerve of the eye of the subject.
61. The method of any one of clauses 58-60, wherein the subject has received a radiation treatment for a disease selected from the group consisting of an intraocular tumor a head tumor, a neck tumor, or a combination thereof, resulting in delayed onset of the disease.
62. The method of clause 61, wherein the administering comprises administering the formulation comprising the effective amount of the pro-angiogenic factor and/or the vascular maturation factor about 1-26 weeks after the subject receives the radiation treatment.
63. A method for treating an ocular disease in a subject in need thereof, comprising:
   administering a formulation to the subject, wherein the formulation comprises a therapeutically effective amount of a pro-angiogenic factor and/or a vascular maturation factor.
64. The method of clause 63, thereby the progression of the macular disease or ocular disease in the subject is treated or slowed.
65. A method for treating an ocular disease in a subject in need thereof, comprising:
   causing a level of a pro-angiogenic factor in the choroid or retina of an eye of the subject to increase; and
   administering a therapeutically effective amount of a vascular maturation factor to the subject.
66. The method of clause 65, thereby the progression of the ocular disease in the subject is treated or slowed.
67. The method of any one of clauses 65-66, wherein causing the level of the pro-angiogenic factor in the choroid of the eye of the subject to increase comprises discontinuing or reducing the administration frequency of an antagonist of a second pro-angiogenic factor.
68. The method of clause 67, wherein the pro-angiogenic factor and the second pro-angiogenic factor are identical.
69. The method of any one of clauses 67-68, wherein the antagonist of the second pro-angiogenic factor comprises an antibody targeting the second pro-angiogenic factor, or a fragment thereof.
70. The method of clause 65, wherein causing the level of the pro-angiogenic factor in the choroid of the eye of the subject to increase comprises administering a therapeutically effective amount of the pro-angiogenic factor to the subject.
71. The method of any one of clauses 63-70, wherein the ocular disease is wet age-related macular degeneration (AMD), polypoidal choroidal vasculopathy (PCV), degenerate (pathologic) myopia, or a combination thereof.
72. The method of any one of clauses 63-70, wherein the ocular disease is associated with, or characterized by, choroidal hypoperfusion, choroidal neovascularization (CNV), macular atrophy, or a combination thereof.
73. The method of any one of clauses 63-70, wherein the ocular disease is dry age-related macular degeneration (AMD), diabetic macular edema, macular edema from retinal vein occlusion, diabetic retinopathy, retinal vein occlusion, retinopathy of prematurity, retinal neovascularization, optic nerve neovascularization, anterior segment neovascularization, familial exudative vitreoretinopathy, radiation retinopathy, radiation optic neuropathy, sickle cell disease, or a combination thereof.
74. The method of any one of clauses 63-70, wherein the ocular disease is associated with, or characterized by, retinal hypoperfusion, retinal ischemia, optic nerve ischemia, or a combination thereof.
75. The method of any one of clauses 1-74, comprising determining the severity of the ocular disease in the subject or the rate of progression of the ocular disease in the subject.
76. The method of any one of clauses 1-75, comprising identifying the subject as needing increased choroidal perfusion, as needing non-leaking or minimally-leaking choroidal revascularization, as needing increased retinal perfusion, as needing non-leaking or minimally-leaking retinal revascularization, as suffering from the ocular disease, or a combination thereof.
77. The method of any one of clauses 1-75, wherein the subject is known to need increased choroidal perfusion, non-leaking or minimally-leaking choroidal revascularization, increased retinal perfusion, non-leaking or minimally-leaking retinal revascularization, or have the ocular disease.
78. The method of any one of clauses 1-77, wherein the pro-angiogenic factor is a recombinant pro-angiogenic factor, a mutant the pro-angiogenic factor, or a combination thereof.
79. The method of any one of clauses 1-78, wherein the pro-angiogenic factor is vascular endothelial growth factor (VEGF), angiopoietin-2 (Ang-2), or a combination thereof.
80. The method of clause 79, wherein the VEGF is VEGF-A, VEGF-B, VEGF-C, VEGF-D, placental growth factor (PIGF), or a combination thereof.
81. The method of any one of clauses 1-80, wherein the vascular maturation factor is a recombinant vascular maturation factor, a mutation vascular maturation factor, or a combination thereof.
82. The method of any one of clauses 1-81, wherein the vascular maturation factor is platelet-derived growth factor (PDGF), angiopoietin-1 (Ang-1), or a combination thereof.
83. The method of any one of clauses 1-81, wherein the vascular maturation factor is PDGF subunit A, PDGF subunit B, PDGF subunit C, PDGF subunit D, or a combination thereof.
84. The method of any one of clauses 1-83, wherein exudation or neovascularization in the choroid or retina of the eye of the subject is reduced.
85. The method of clause 84, comprising determining whether the exudation in the choroid or retina of the eye of the subject is reduced using optical coherence tomography (OCT).
86. The method of any one of clauses 84-85, comprising determining whether the neovascularization in the choroid or retina of the eye of the subject is reduced using optical coherence tomography angiography (OCT-A), fluorescein angiography (FA), indocyanine green (ICG) angiography, or a combination thereof.
87. The method of any one of clauses 1-86, thereby non-leaking or minimally-leaking choroidal perfusion and/or non-leaking and/or minimally-leaking retinal perfusion increases in the subject by at least 5%.
88. The method of any one of clauses 1-87, thereby new non-leaking or minimally-leaking blood vessels are formed in the choroid or the retina of the eye of the subject.
89. The method of clause 88, comprising determining the formation of new blood vessels using optical coherence tomography angiography (OCT-A).
90. The method of any one of clauses 88-89, comprising determining minimal or no exudation from the new blood vessels is minimal using optical coherence tomography (OCT), which indicates the new blood vessels formed are non-leaking or minimally-leaking.
91. The method of any one of clauses 88-90, wherein the new non-leaking or minimally-leaking blood vessels formed in the choroid or the retina of the eye of the subject cover at least 5% of the macular region of the eye of the subject.
92. The method of any one of clauses 88-91, wherein the new non-leaking or minimally-leaking blood vessels formed in the choroid or retina of the eye of the subject cover at least 5% of the peripheral choroid or peripheral retina of the eye of the subject.
93. The method of any one of clauses 1-92, wherein the visual acuity of the subject stabilizes or improves.
94. The method of any one of clauses 1-93, wherein choroidal hypoxia and/or retinal hypoxia is mitigated in the subject.
95. The method of any one of clauses 1-94, thereby a hypoxia inducible factor (HIF)-mediated blinding complication in the subject is mitigated.
96. The method of clause 95, wherein the HIF-mediated visual loss complication comprises choroidal neovascularization (CNV), retinal neovascularization, macular edema, or a combination thereof.
97. The method of any one of clauses 1-96, thereby retinal edema, subretinal fluid, or both, are reduced.
98. The method of any one of clauses 1-97, thereby leaky choroidal neovascularization is mitigated in the subject.
99. The method of any one of clauses 1-98, thereby macular atrophy, geographic atrophy (GA), or both are mitigated in the subject.
100. The method of any one of clauses 1-99, wherein the administering comprises administering the formulation intravitreally.
101. The method of any one of clauses 1-99, wherein the administering comprises administering the formulation subretinally.
102. The method of any one of clauses 1-99, wherein the administering comprises administering the formulation to the suprachoroidal space of an eye of the subject.
103. The method of any one of clauses 101-102, wherein the administering comprises administering the formulation to the macular region of an eye of the subject.
104. The method of any one of clauses 101-102, wherein the administering comprises administering the formulation to one or more retinal or choroidal regions, of the eye of the subject, with reduced perfusion.
105. The method of any one of clauses 1-104, wherein the administering comprises administering the formulation to the subject about once every week to about once every year.
106. The method of any one of clauses 1-105, wherein the administering comprises administering the formulation over about three months to about 12 months.
107. The method of any one of clauses 1-105, further comprising:
   using optical coherence tomography angiography (OCT-A) and optical coherence tomography (OCT) to determine choroidal or retinal revascularization or non-leaking or minimally-leaking choroidal or retinal revascularization in the subject, respectively; and
   if adequate, discontinuing temporarily or permanently administering the formulation to the subject.
108. The method of any one of clauses 1-105, further comprising:
   using optical coherence tomography angiography (OCT-A) and optical coherence tomography (OCT) to determine choroidal or retinal revascularization or non-leaking or minimally-leaking choroidal or retinal revascularization in the subject, respectively; and
   if inadequate, continuing administering the formulation to the subject.
109. The method of any one of clauses 1-105, further comprising:
   using optical coherence tomography angiography (OCT-A) and optical coherence tomography (OCT) to determine choroidal or retinal revascularization or non-leaking or minimally-leaking choroidal or retinal revascularization in the subject, respectively; and
   if excessive, administering an antagonist of a second pro-angiogenic factor to the subj ect.
110. The method of clause 109, wherein the antagonist of the second pro-angiogenic factor comprises an antibody targeting the second pro-angiogenic factor.
111. The method of any one of clauses 107-110, wherein the determining comprises performing an ocular examination or sequential ocular examinations.
112. The method of clause 111, wherein the sequential ocular examinations comprise visual acuity assessment, a fundus auto-fluorescence (FAF) examination, an optical coherence tomography (OCT) examination, an optical coherence tomography angiography (OCT-A) examination, a fluorescein angiography (FA) examination, indocyanine green (ICG) angiography examination, or a combination thereof.
113. The method of any one of clauses 1-112, further comprising:
   determining vascular maturation in the subject using optical coherence tomography angiography (OCT), fluorescein angiography (FA), indocyanine green (ICG) angiography, or a combination thereof; and
   if inadequate, administering the vascular maturation factor to the subject.
114. The method of clause 113, the determining comprises determining the extent of vascular maturation using optical coherence tomography (OCT) or fluorescein and indocyanine green (ICG) angiography.
115. The method of any one of clauses 113-114, further comprising: using optical coherence tomography angiography (OCT-A) to determine choroidal revascularization in the subj ect.
116. The method of any one of clauses 1-115, wherein the therapeutically effective amount of the pro-angiogenic factor is about 0.01 mg to about 100 mg per administering.
117. The method of any one of clauses 1-116, wherein the therapeutically effective amount of the vascular maturation factor is about 0.01 mg to about 100 mg of the vascular maturation factor per administering.
118. The method of any one of clauses 1-117, wherein the formulation comprises about 0.001 mg/ml to about 10 mg/ml of the pro-angiogenic factor.
119. The method of any one of clauses 1-118, wherein the formulation comprises about 0.001 mg/ml to about 10 mg/ml of the vascular maturation factor.
120. The method of any one of clauses 1-119, wherein the formulation comprises a sustained release formulation of the pro-angiogenic factor and the vascular maturation factor.
121. A composition comprising a pro-angiogenic factor and/or a vascular maturation factor for use in increasing non-leaking or minimally-leaking choroidal perfusion or non-leaking or minimally-leaking retinal perfusion in a subject in need thereof.
122. A composition comprising a pro-angiogenic factor and/or a vascular maturation factor for use in the treatment of an ocular disease associated with, or characterized by, choroidal hypoperfusion or retinal hypoperfusion in a subject in need thereof.
123. A composition comprising an angiogenesis factor for use in increasing non-leaking or minimally-leaking choroidal perfusion or non-leaking or minimally-leaking retinal perfusion in a subject in need thereof.
124. A composition comprising an angiogenesis for use in the treatment of an ocular disease associated with, or characterized by, choroidal hypoperfusion or retinal hypoperfusion in a subject in need thereof.
125. The composition of any one of clauses 123-124, wherein the angiogenesis factor is a pro-angiogenic factor and/or a vascular maturation factor.
126. The composition of any one of clauses 121-125, wherein the pro-angiogenic factor is a recombinant pro-angiogenic factor, a mutation pro-angiogenic factor, or a combination thereof.
127. The composition of any one of clauses 121-126, wherein the pro-angiogenic factor is vascular endothelial growth factor (VEGF), angiopoietin-2 (Ang-2), or a combination thereof.
128. The composition of clause 127, wherein the VEGF is VEGF-A, VEGF-B, VEGF-C, VEGF-D, placental growth factor (PIGF), or a combination thereof.
129. The composition of any one of clauses 121-128, wherein the vascular maturation factor is a recombinant vascular maturation factor, a mutant vascular maturation factor, or a combination thereof.
130. The composition of any one of clauses 121-129, wherein the vascular maturation factor is platelet-derived growth factor (PDGF), angiopoietin-1 (Ang-1), or a combination thereof.
131. The composition of any one of clauses 121-130, wherein the vascular maturation factor is PDGF subunit A, PDGF subunit B, PDGF subunit C, PDGF subunit D, or a combination thereof.
132. The composition of any one of clauses 121-131, wherein non-leaking or minimally-leaking choroidal perfusion in the subject is increased after the composition is administered to the subject.
133. The composition of any one of clauses 121-132, wherein non-leaking or minimally-leaking choroidal revascularization in the subject is promoted after the composition is administered to the subject.
134. The composition of any one of clauses 121-133, wherein macular flow voids are reduced, hypoxia in the outer retina and retinal pigment epithelium (RPE) of the eye of the subject is reduced, and/or ischemia in the outer retina and RPE of the eye of the subject is reduced after the composition is administered to the subject.
135. The composition of any one of clauses 121-134, wherein non-leaking or minimally-leaking choroidal perfusion in the subject is increased after the composition is administered to the subject.
136. The composition of any one of clauses 121-135, wherein non-leaking or minimally-leaking retinal perfusion in the subject is increased after the composition is administered to the subject.
137. The composition of any one of clauses 121-136, wherein non-leaking or minimally-leaking retinal revascularization in the subject is promoted after the composition is administered to the subject.
138. The composition of any one of clauses 121-137, wherein hypoxia in the retina of the eye of the subject is reduced, and/or ischemia in the retina of the eye of the subject is reduced after the composition is administered to the subject.
139. The composition of any one of clauses 121-138, wherein non-leaking or minimally-leaking retinal perfusion in the subject is increased after the composition is administered to the subject.
140. The composition of any one of clauses 121-139, wherein exudation or neovascularization in the choroid or retina of the eye of the subject is reduced after the composition is administered to the subject.
141. The composition of any one of clauses 121-140, wherein non-leaking or minimally-leaking choroidal perfusion and/or non-leaking and/or minimally-leaking retinal perfusion in the subject increases by at least 5% after the composition is administered to the subj ect.
142. The composition of any one of clauses 121-141, wherein new non-leaking or minimally-leaking blood vessels are formed in the choroid or the retina of the eye of the subject after the composition is administered to the subject.
143. The composition of any one of clauses 121-142, wherein retinal edema, subretinal fluid, or both, in the subject are reduced after the composition is administered to the subject.
144. The composition of any one of clauses 121-143, wherein leaky choroidal neovascularization in the subject is mitigated after the composition is administered to the subject.
145. The composition of any one of clauses 121-144, wherein macular atrophy, geographic atrophy (GA), or both in the subject are mitigated after the composition is administered to the subject.
146. The composition of any one of clauses 121-145, wherein the subject has a disease selected from the group consisting of dry age-related macular degeneration (AMD) and/or geographic atrophy (GA), or a combination thereof
147. The composition of clause 146, wherein the composition, after being administered to the subject, results in reversing, halting, or slowing of the progression of the disease in the subj ect.
148. The composition of clause 147, wherein the reversing, halting, or slowing of the progression of the disease is mediated by increased choroidal perfusion and/or non-leaking or minimally-leaking choroidal revascularization.
149. The composition of any one of clauses 121-145, wherein the subject has a disease selected from the group consisting of wet age-related macular degeneration (AMD), choroidal neovascularization (CNV), polypoidal choroidal vasculopathy, degenerative (pathologic) myopia, giant cell arteritis, or a combination thereof.
150. The composition of clause 149, wherein the composition, after being administered to the subject, results in reversing, halting, or slowing the progression of the disease in the subj ect.
151. The composition of clause 150, wherein the reversing, halting, or slowing of the progression of the disease is mediated by increased choroidal perfusion and/or non-leaking or minimally-leaking choroidal revascularization.
152. The composition of any one of clauses 121-145, wherein the subject has a disease selected from the group consisting of diabetic macular edema, macular edema from retinal vein occlusion, diabetic retinopathy, retinal vein occlusion, retinopathy of prematurity, retinal neovascularization in diabetes, optic nerve neovascularization in diabetes, familial exudative vitreoretinopathy, sickle cell disease, or a combination thereof.
153. The composition of clause 152, wherein the composition, after being administered to the subject, results in halting or slowing of the progression of the disease in the subject.
154. The composition of clause 153, wherein the halting or slowing of the progression of the disease is mediated by increased non-leaking or minimally-leaking retinal perfusion and/or non-leaking or minimally-leaking retinal revascularization.
155. The composition of any one of clauses 121-145, wherein the subject has a disease selected from the group consisting of radiation retinopathy, radiation optic neuropathy, or a combination thereof.
156. The composition of clause 155, wherein the composition, after being administered to the subject, results in reversing, halting, or slowing of the progression of the disease in the subj ect.
157. The composition of clause 156, wherein the reversing, halting, or slowing of the progression of the disease is mediated by increased non-leaking or minimally-leaking retinal perfusion, non-leaking or minimally-leaking retinal revascularization and/or non-leaking or minimally-leaking revascularization of the optic nerve of the eye of the subject.
158. The composition of any one of clauses 155-157, wherein the subject has received a radiation treatment for a disease selected from the group consisting of an intraocular tumor a head tumor, a neck tumor, or a combination thereof, resulting in delayed onset of the disease.
159. The composition of clause 158, wherein the administration of the composition to the subject occurs about 1-26 weeks after the subject receives the radiation treatment.
160. The composition of any one of clauses 121-145, wherein the progression of the ocular disease in the subject is treated or slowed after the composition is administered to the subj ect.
161. The composition of any one of clauses 121-145, wherein a hypoxia inducible factor (HIF)-mediated blinding complication in the subject is mitigated after the composition is administered to the subject.
162. The composition of clause 161, wherein the HIF-mediated visual loss complication comprises choroidal neovascularization (CNV), retinal neovascularization, macular edema, or a combination thereof.
163. The composition of any one of clauses 121-162, wherein the composition is formulated for intravitreal administration.
164. The composition of any one of clauses 121-162, wherein the composition is formulated for subretinal administration.
165. The composition of any one of clauses 121-162, wherein the composition is formulated for administration to the suprachoroidal space of an eye of the subject.
166. The composition of any one of clauses 164-165, wherein the composition is formulated for administration to the macular region of an eye of the subject.
167. The composition of any one of clauses 164-165, the composition is formulated for administration to one or more retinal or choroidal regions, of the eye of the subject, with reduced perfusion.
168. The composition of any one of clauses 121-167, wherein the composition is formulated for administration to the subject about once every week to about once every year.
169. The composition of any one of clauses 121-168, wherein the composition is formulated for administration to the subject over about three months to about 12 months.
170. The composition of any one of clauses 121-169, wherein the formulation comprises about 0.001 mg/ml to about 10 mg/ml of the pro-angiogenic factor.
171. The composition of any one of clauses 121-170, wherein the formulation comprises about 0.001 mg/ml to about 10 mg/ml of the vascular maturation factor.
172. A kit comprising:
   a formulation comprising a pro-angiogenic factor and/or a vascular maturation factor; and
   a label indicating that the formulation is for increasing choroidal perfusion or retinal perfusion.
173. A kit comprising:
   a formulation comprising a pro-angiogenic factor and/or a vascular maturation factor; and
   a label indicating that the formulation is for treating an ocular disease associated with, or characterized by, choroidal hypoperfusion or retinal hypoperfusion.
174. A kit comprising:
   a formulation comprising an angiogenesis factor; and
   a label indicating that the formulation is for increasing choroidal perfusion or retinal perfusion.
175. A kit comprising:
   a formulation comprising an angiogenesis factor; and
   a label indicating that the formulation is for treating an ocular disease associated with, or characterized by, choroidal hypoperfusion or retinal hypoperfusion.
176. The kit of any one of clauses 174-175, wherein the angiogenesis factor is a pro-angiogenic factor and/or a vascular maturation factor.
177. The kit of any one of clauses 172-176, wherein the pro-angiogenic factor is a recombinant pro-angiogenic factor, a mutant pro-angiogenic factor, or a combination thereof.
178. The kit of any one of clauses 172-177, wherein the pro-angiogenic factor is vascular endothelial growth factor (VEGF), angiopoietin-2 (Ang-2), or a combination thereof.
179. The kit of clause 178, wherein the VEGF is VEGF-A, VEGF-B, VEGF-C, VEGF-D, placental growth factor (PIGF), or a combination thereof.
180. The kit of any one of clauses 172-179, wherein the vascular maturation factor is a recombinant vascular maturation factor, a mutant vascular maturation factor, or a combination thereof.
181. The kit of any one of clauses 172-180, wherein the vascular maturation factor is platelet-derived growth factor (PDGF), angiopoietin-1 (Ang-1), or a combination thereof.
182. The kit of any one of clauses 172-181, wherein the vascular maturation factor is PDGF subunit A, PDGF subunit B, PDGF subunit C, PDGF subunit D, or a combination thereof.
183. The kit of any one of clauses 172-182, wherein non-leaking or minimally-leaking choroidal perfusion in the subject is increased after the formulation is administered to the subject.
184. The kit of any one of clauses 172-183, wherein non-leaking or minimally-leaking choroidal revascularization in the subject is promoted after the formulation is administered to the subj ect.
185. The kit of any one of clauses 172-184, wherein macular flow voids are reduced, hypoxia in the outer retina and retinal pigment epithelium (RPE) of the eye of the subject is reduced, and/or ischemia in the outer retina and RPE of the eye of the subject is reduced after the formulation is administered to the subject.
186. The kit of any one of clauses 172-185, wherein non-leaking or minimally-leaking choroidal perfusion in the subject is increased after the formulation is administered to the subject.
187. The kit of any one of clauses 172-186, wherein the label indicates the formulation is for treating a disease selected from the group consisting of dry age-related macular degeneration (AMD) and/or geographic atrophy (GA), or a combination thereof.
188. The kit of any one of clauses 172-186, wherein the label indicates the formulation is for treating a disease selected from the group consisting of wet age-related macular degeneration (AMD), choroidal neovascularization (CNV), polypoidal choroidal vasculopathy, degenerative (pathologic) myopia, giant cell arteritis, or a combination thereof.
189. The kit of any one of clauses 172-186, wherein the label indicates the formation is for treating a disease selected from the group consisting of diabetic macular edema, macular edema from retinal vein occlusion, diabetic retinopathy, retinal vein occlusion, retinopathy of prematurity, retinal neovascularization in diabetes, optic nerve neovascularization in diabetes, familial exudative vitreoretinopathy, sickle cell disease, or a combination thereof.
190. The kit of clause 189, wherein non-leaking or minimally-leaking retinal perfusion in the subject is increased after the formulation is administered to the subject.
191. The kit of any one of clauses 189-190, wherein non-leaking or minimally-leaking retinal revascularization in the subject is promoted after the formulation is administered to the subj ect.
192. The kit of any one of clauses 189-191, wherein hypoxia in the retina of the eye of the subject is reduced, and/or ischemia in the retina of the eye of the subject is reduced after the formulation is administered to the subject.
193. The kit of any one of clauses 189-192, wherein non-leaking or minimally-leaking retinal perfusion in the subject is increased after the formulation is administered to the subject.
194. The kit of any one of clauses 172-186, wherein the label indicates the formulation is for treating a disease selected from the group consisting of radiation retinopathy, radiation optic neuropathy, or a combination thereof.
195. The kit of clause 194, wherein the label indicates the formulation is for treating the subject after the subject receives a radiation treatment for a disease selected from the group consisting of an intraocular tumor a head tumor, a neck tumor, or a combination thereof, resulting in delayed onset of the disease.
196. The kit of clause 195, wherein the label indicates the administration of the formulation to the subject occurs about 1-26 weeks after the subject receives the radiation treatment.
197. The kit of any one of clauses 172-186, wherein the label indicates the formulation is for treating a hypoxia inducible factor (HIF)-mediated blinding complication.
198. The kit of clause 197, wherein the HIF-mediated visual loss complication comprises choroidal neovascularization (CNV), retinal neovascularization, macular edema, or a combination thereof.
199. The kit of any one of clauses 172-198, wherein exudation or neovascularization in the choroid or retina of the eye of the subject is reduced after the formulation is administered to the subject.
200. The kit of any one of clauses 172-199, wherein non-leaking or minimally-leaking choroidal perfusion and/or non-leaking and/or minimally-leaking retinal perfusion in the subject increases by at least 5% after the formulation is administered to the subject.
201. The kit of any one of clauses 172-200, wherein new non-leaking or minimally-leaking blood vessels are formed in the choroid or the retina of the eye of the subject after the formulation is administered to the subject.
202. The kit of any one of clauses 172-201, wherein retinal edema, subretinal fluid, or both, in the subject are reduced after the formulation is administered to the subject.
203. The kit of any one of clauses 172-202, wherein leaky choroidal neovascularization in the subject is mitigated after the formulation is administered to the subject.
204. The kit of any one of clauses 172-203, wherein macular atrophy, geographic atrophy (GA), or both in the subject are mitigated after the formulation is administered to the subj ect.
205. The kit of any one of clauses 172-204, wherein the composition is formulated for intravitreal administration.
206. The kit of any one of clauses 172-204, wherein the composition is formulated for subretinal administration.
207. The kit of any one of clauses 172-204, wherein the composition is formulated for administration to the suprachoroidal space of an eye of the subject.
208. The kit of any one of clauses 164-165, wherein the composition is formulated for administration to the macular region of an eye of the subject.
209. The kit of any one of clauses 164-165, the composition is formulated for administration to one or more retinal or choroidal regions, of the eye of the subject, with reduced perfusion.
210. The kit of any one of clauses 172-167, wherein the composition is formulated for administration to the subject about once every week to about once every year.
211. The kit of any one of clauses 172-168, wherein the composition is formulated for administration to the subject over about three months to about 12 months.
212. The kit of any one of clauses 172-182, wherein the formulation comprises about 0.001 mg/ml to about 10 mg/ml of the pro-angiogenic factor.
213. The kit of any one of clauses 172-212, wherein the formulation comprises about 0.001 mg/ml to about 10 mg/ml of the vascular maturation factor.

## Claims

1. An angiogenesis factor comprising leukemia inhibitory factor (LIF) for use in a method for increasing choroidal perfusion in a subject in need thereof, the method comprising:
administering a formulation comprising a therapeutically effective amount of the angiogenesis factor to the subject in need of increased choroidal perfusion, thereby increasing non-leaking or minimally-leaking choroidal perfusion in the subject; and
determining an extent of choroidal perfusion in the subject to be inadequate and continuing to administer the formulation comprising the effective amount of the angiogenesis factor, wherein the determining comprises performing sequential ocular examinations comprising an optical coherence tomography angiography (OCT- A) examination.

2. The angiogenesis factor of claim 1, wherein increased non-leaking or minimally-leaking choroidal perfusion comprises increased choriocapillaris perfusion.

3. The angiogenesis factor of claim 1 or 2, thereby non-leaking or minimally-leaking choroidal revascularization in the subject is promoted.

4. The angiogenesis factor of any one of the preceding claims, wherein the method further comprises: prior to the administering, determining the subject is in need of increased choroidal perfusion or non-leaking or minimally-leaking choroidal revascularization with an ocular examination.

5. The angiogenesis factor of any one of the preceding claims, wherein the sequential ocular examinations comprise visual acuity assessment, a fundus auto-fluorescence (FAF) examination, an optical coherence tomography (OCT) examination, an optical coherence tomography angiography (OCT- A) examination, a fluorescein angiography (FA) examination, an indocyanine green (ICG) angiography examination, or a combination thereof.

6. The angiogenesis factor of any one of the preceding claims, wherein the subject has a disease selected from the group consisting of dry age-related macular degeneration (AMD), geographic atrophy (GA), and a combination thereof.

7. The angiogenesis factor of any one of the preceding claims, wherein the subject has a disease selected from the group consisting of wet age-related macular degeneration (AMD), choroidal neovascularization (CNV), polypoidal choroidal vasculopathy, degenerative (pathologic) myopia, and a combination thereof.

8. The angiogenesis factor of any one of the preceding claims, thereby a progression of the disease is reversed, halted, or slowed, wherein the reversing, halting, or slowing of the progression of the disease is mediated by increased choroidal perfusion and/or non-leaking or minimally-leaking choroidal revascularization.

9. The angiogenesis factor of any one of the preceding claims, thereby macular flow voids are reduced, hypoxia in the outer retina and retinal pigment epithelium (RPE) of the eye of the subject is reduced, and/or ischemia in the outer retina and RPE of the eye of the subject is reduced.

10. The angiogenesis factor of any one of the preceding claims, wherein a concentration of the angiogenesis factor in the formulation is at least about 0.01 mg/mL.

11. The angiogenesis factor of any one of the preceding claims, wherein the method further comprises:
using optical coherence tomography angiography (OCT- A) and optical coherence tomography (OCT) to determine choroidal or retinal revascularization or non-leaking or minimally-leaking choroidal or retinal revascularization in the subject, respectively; and
if inadequate, continuing administering the formulation to the subject.

12. The angiogenesis factor of any one of the preceding claims, wherein the method further comprises:
using optical coherence tomography angiography (OCT- A) and optical coherence tomography (OCT) to determine choroidal or retinal revascularization or non-leaking or minimally-leaking choroidal or retinal revascularization in the subject, respectively; and
if excessive, administering an antagonist of a pro-angiogenic factor to the subject.

13. The angiogenesis factor of claim 12, wherein the antagonist of the pro-angiogenic factor comprises an antibody targeting the second pro-angiogenic factor.

14. The angiogenesis factor of any one of the preceding claims, wherein the method further comprises:
using optical coherence tomography angiography (OCT- A) and optical coherence tomography (OCT) to determine choroidal or retinal revascularization or non-leaking or minimally-leaking choroidal or retinal revascularization in the subject, respectively; and
if adequate, discontinuing temporarily or permanently administering the formulation to the subject.

15. The angiogenesis factor of any one of the preceding claims, wherein the method further comprises:
determining vascular maturation in the subject using optical coherence tomography angiography (OCT), fluorescein angiography (FA), indocyanine green (ICG) angiography, or a combination thereof; and
if inadequate, administering a vascular maturation factor to the subject.
